# EUROPEAN PATENT APPLICATION

(11) **EP 4 728 998 A1**
(43) Date of publication of application: **22.04.2026**
(21) Application number: 25209631.8
(22) Date of filing: 18.10.2025
(51) Int. Cl.: A61B 17/04

(54) **PROBE DEVICE AND GRASPING DEVICE**

(30) Priority: 18.10.2024 US 202463708833 P; 10.06.2025 US 202563821112 P; 17.10.2025 US 202519361663
(71) Applicant: LSI Solutions, Inc., Victor, NY 14564 (US)
(72) Inventor: SAUER, Jude S., Pittsford, 14534 (US)
(74) Representative: Flügel Preissner Schober Seidel

(57) **Abstract**

A grasping device (10, 200, 300, 400, 500) for use in a surgical procedure, the grasping device (10, 200, 300, 400, 500) comprising: a housing portion (12); an actuator lever (16) pivotably coupled to a first portion (15, 18a, 19a, 24a, 58) of the housing portion (12) and displaceable between a first lever position to a second lever position; a shaft (18) that extends from a proximal end (20, 34, 38, 41, 42, 46, 54, 62, 66, 68, 74, 82, 90, 98, 438, 462) to a distal end (22, 36, 40, 42, 48, 56, 64, 68, 76, 84, 92, 101, 440, 464) along a shaft axis (19, 24); an elongated push rod (36, 436) extending from a proximal end (20, 34, 38, 41, 42, 46, 54, 62, 66, 68, 74, 82, 90, 98, 438, 462) to a distal end (22, 36, 40, 42, 48, 56, 64, 68, 76, 84, 92, 101, 440, 464); and a jaw assembly (60, 62) disposed at or adjacent to the distal end (22, 36, 40, 42, 48, 56, 64, 68, 76, 84, 92, 101, 440, 464) of the shaft (18). The jaw assembly (60, 62) comprises a first jaw (62, 64, 462) fixedly coupled to a portion (80, 88, 96) of the shaft (18) at or adjacent to the distal end (22, 36, 40, 42, 48, 56, 64, 68, 76, 84, 92, 101, 440, 464) of the shaft (18); and a second jaw (64) coupled to the distal end (22, 36, 40, 42, 48, 56, 64, 68, 76, 84, 92, 101, 440, 464) of the push rod (36, 436) such that the second jaw (64) is displaceable relative to the first jaw (62, 64, 462) and such that in the first rod position, the jaw assembly (60, 62) is in a first position and in the second rod position, the jaw assembly (60, 62) in in a second position.

## Description

### CROSS-REFERENCE TO RELATED APPLICATIONS

This application claims the benefit of U.S. Provisional Patent Application No. 63/708,833, filed October 18, 2024, U.S. Provisional Patent Application No. 63/821,112, filed June 10, 2025, and U.S. Non-Provisional Patent Application No. 19/361,663, filed October 17, 2025, the contents of each of which is incorporated by reference herein in its entirety.

### FIELD OF THE INVENTION

The claimed invention relates to surgical devices, and more specifically to a surgical probe device and a surgical grasping device.

### BACKGROUND OF THE INVENTION

In spinal surgery, incisions through the dura mater are a fundamental aspect of many procedures aimed at accessing the intradural space to address pathologies affecting the spinal cord, nerve roots, or surrounding structures. The dura mater, the outermost meningeal layer enveloping the central nervous system, acts as a protective barrier that contains cerebrospinal fluid (CSF), providing mechanical support and cushioning against trauma. Such dural incisions are routinely performed in operations like laminectomies for spinal decompression, tumor resections, discectomies, or repairs of congenital anomalies such as tethered cord syndrome. These incisions enable surgeons to directly visualize and manipulate neural elements, facilitating precise interventions that can alleviate pain, restore neurological function, or prevent further deterioration. Historically, advancements in microsurgical techniques and imaging have refined the approach to dural incisions, minimizing unnecessary tissue disruption while ensuring adequate exposure for effective treatment.

Despite these advancements, suturing the dural incision presents notable challenges and limitations that can compromise postoperative outcomes. Achieving a watertight closure is critical to prevent CSF leakage, yet the dura's thin, delicate nature often leads to difficulties in handling and approximation, particularly in cases involving inflammation, scarring from prior surgeries, or age-related tissue fragility. Traditional suturing methods are time-consuming and require high technical proficiency within the confined spinal canal, increasing the risk of inadvertent neural injury or prolonged operative duration. Complications arising from inadequate suturing include persistent CSF fistulas, which may result in low-pressure headaches, infections, pseudomeningocele formation, or even meningitis, necessitating additional interventions like lumbar drains or revisions. These issues highlight the need for improved closure techniques that enhance reliability, reduce surgical time, and minimize patient morbidity.

### SUMMARY OF THE INVENTION

The invention is set out in the appended claims.

### BRIEF DESCRIPTION OF THE DRAWINGS

Figs. 1A to 1E are various views of an embodiment of a probe device;
Figs. 2A to 2D are various views of a tip portion of the embodiment of the probe device of Figs. 1A to 1E;
Figs. 3A and 3B are cross-sectional views of the embodiment of the probe device of Figs. 1A to 1E taken along the shaft axis of Fig. 1B;
Fig. 4A is a side view of a portion of the probe device of Figs. 1A to 1E with the housing portion omitted for clarity;
Fig. 4B is a detailed view of the side view of Fig. 4A;
Fig. 4C is a perspective view of the detailed view of Fig. 4B;
Fig. 5A is a side view of a probe assembly of the embodiment of the probe device of Figs. 1A to 1E;
Fig. 5B is a view of a distal end of the probe assembly of Fig. 5A;
Fig. 5C is a side view of the probe assembly of Fig. 5A;
Fig. 6 is a perspective view of a portion of the probe device of Figs. 1A to 1E with a lever in an actuated handle position;
Figs. 7A to 7D are various illustration of a method of using the probe device of Figs. 1A to 1E and an embodiment of a grasping device in a procedure;
Fig 8A is a front view of a probe member of the probe device of Figs. 1A to 1E in a first position;
Fig 8B is a front view of a probe member of the probe device of Figs. 1A to 1E in a second position;
Fig. 9 is a partial side view of a portion of an additional embodiment of a probe device with a portion of the housing portion omitted for clarity;
Figs. 10A and 10B are detailed views of the partial side view of the embodiment of the probe device of Fig. 9;
Figs. 11A to 11E are various views of a support body;
Fig. 12 is a partial side view of an embodiment of a grasping device;
Fig. 13 is a side view of a portion of the grasping device of Fig. 12 with a portion of the housing portion omitted for clarity;
Fig. 14 is a side view of the portion of the grasping device of Fig. 12 with the lever in a second lever position;
Fig. 15 is a side view of the grasping device of Fig. 12 with a portion of the housing portion omitted for clarity;
Fig. 16 is a perspective view of a jaw assembly of the grasping device of Fig. 12;
Fig. 17A is a side view of the jaw assembly of the grasping device with the shaft omitted for clarity;
Fig. 17B is a top view of the jaw assembly of the grasping device with the shaft omitted for clarity;
Fig. 17C is a perspective view of the jaw assembly of the grasping device with the shaft omitted for clarity;
Figs. 18A, 18B, 19A, and 19B are various views of the jaw assembly of the grasping device of Fig. 12 in a first jaw position;
Fig. 20 is a side view of the jaw assembly of the grasping device of Fig. 12 in a second jaw position;
Figs 21A to 21D are various views of a first jaw of the jaw assembly;
Figs 22A to 22E are various views of a second jaw of the jaw assembly;
Fig. 23A to 23H are various views of a further embodiment of a grasping device;
Fig. 23I is a side view of the grasping device of Fig. 23A with a portion of the housing portion omitted for clarity;
Figs. 24A to 24C are various views of a portion of a rotation assembly of the grasping device of Fig. 23A with the housing portion omitted for clarity;
; Fig. 24D is a side views of a portion of a rotation assembly of the grasping device of Fig. 23A with a portion of the housing portion omitted for clarity;
Figs. 25A to 25D are various views of a locking lever of a locking assembly of the grasping device of Fig. 23A;

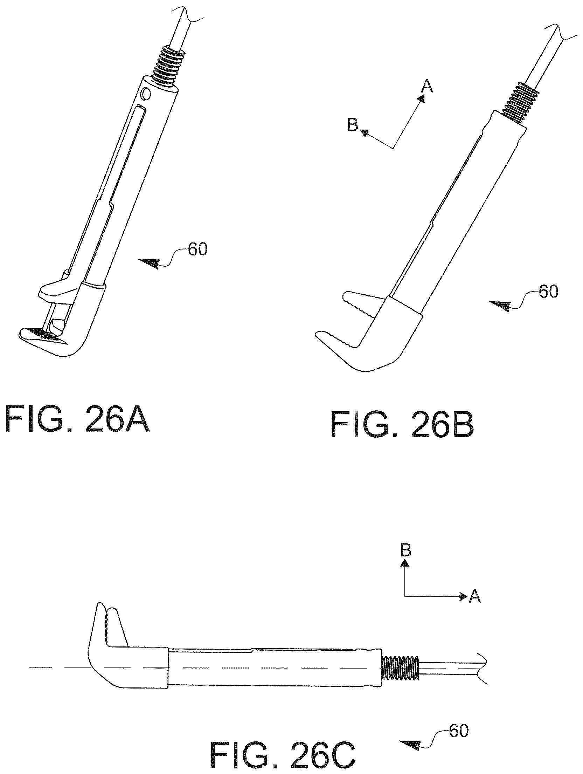

Fig. 26A to 26C are views of the jaw assembly of the grasping device of Fig. 23A;
Fig. 27 is a side view of a second jaw of the jaw assembly of Fig. 28;
Figs. 28A and 28B are cross-sectional views of the jaw assembly of the grasping device of Fig. 23A taken along a first jaw axis;
; Fig. 29A is a side views of a portion of the grasping device of Fig. 23A with the first jaw, the shaft, and a portion of the housing portion omitted for clarity;
Figs. 29B to 29E are detailed views of the transmission rod of the side view of Fig 29A;
Figs. 29F and 29H are cross-sectional views of the transmission rod taken along section line 29F-29F of Fig. 29E;
Fig. 29G is a cross-sectional view of the transmission rod taken along section line 29G-29G of Fig. 29E;
Figs. 30A and 30B are side views of a portion of an embodiment of a rotation assembly of the grasping device of Fig. 23A with a portion of the housing portion omitted for clarity;
Fig. 31 is a bottom view of a rotation knob of the rotation assembly of Fig. 30A;
Figs. 32A to 32D are various views of a further embodiment of a grasping device; and

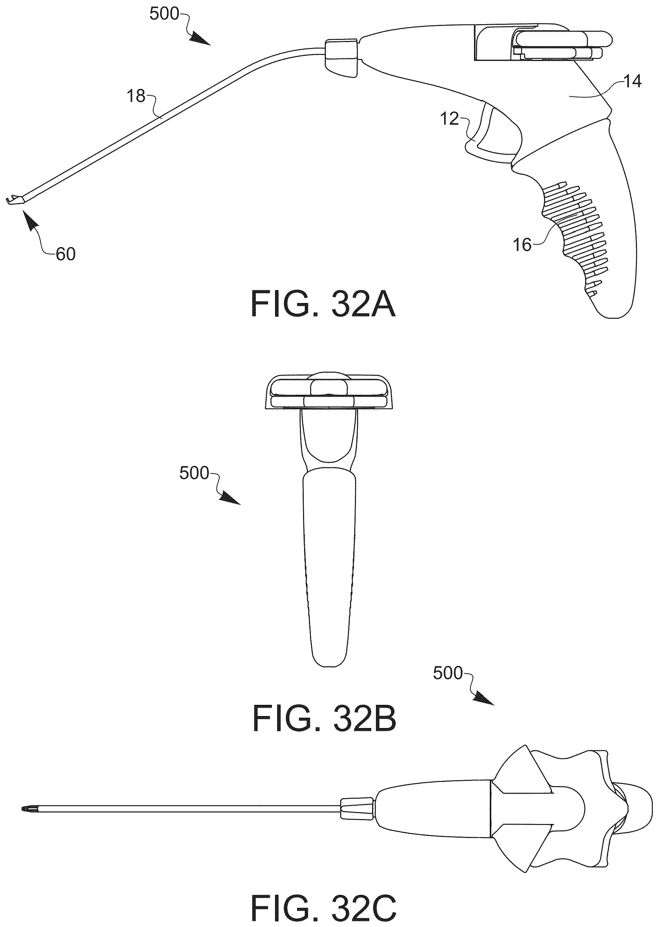

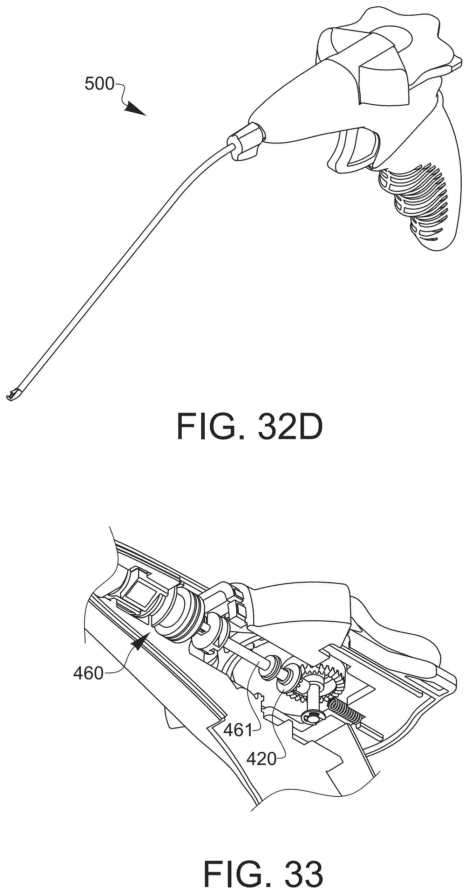

Fig. 32 is a perspective view of a portion of the grasping device of Fig. 32A with a portion of the housing portion omitted for clarity.

### DETAILED DESCRIPTION OF THE INVENTION

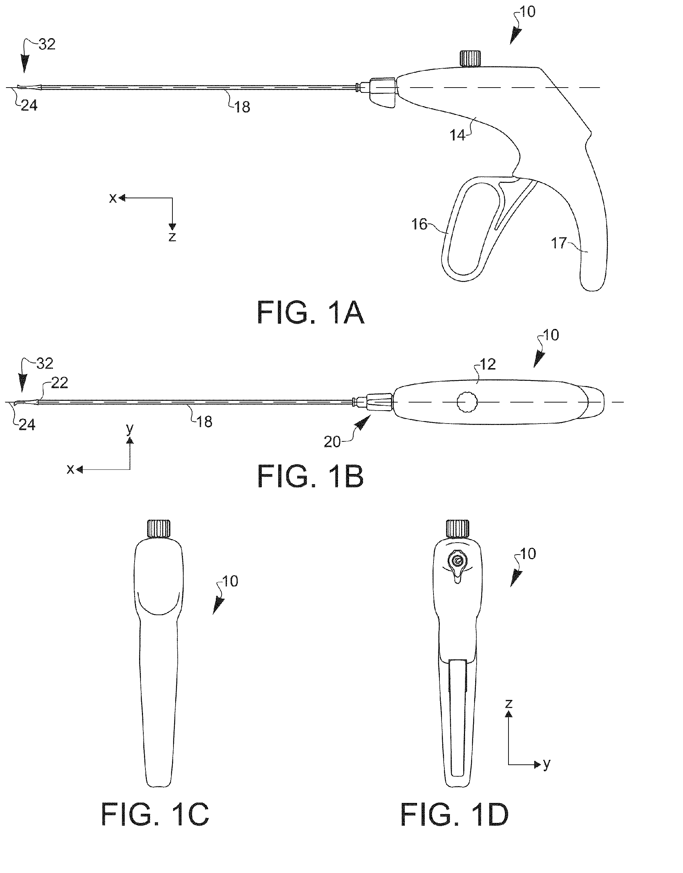

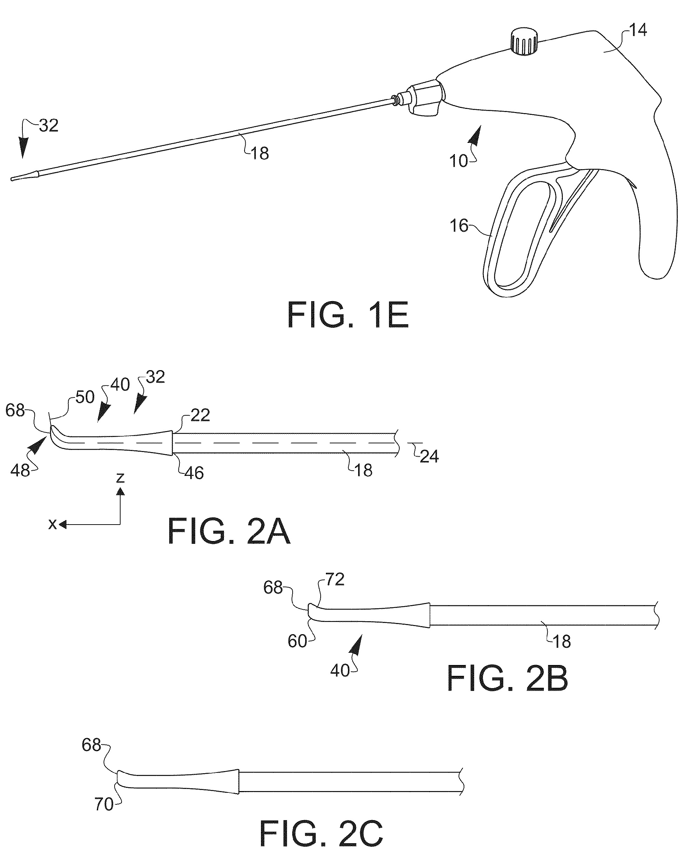

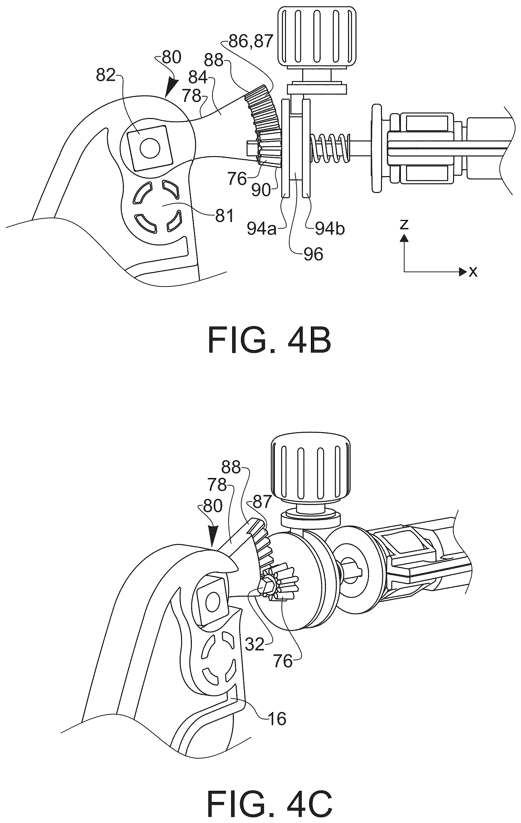

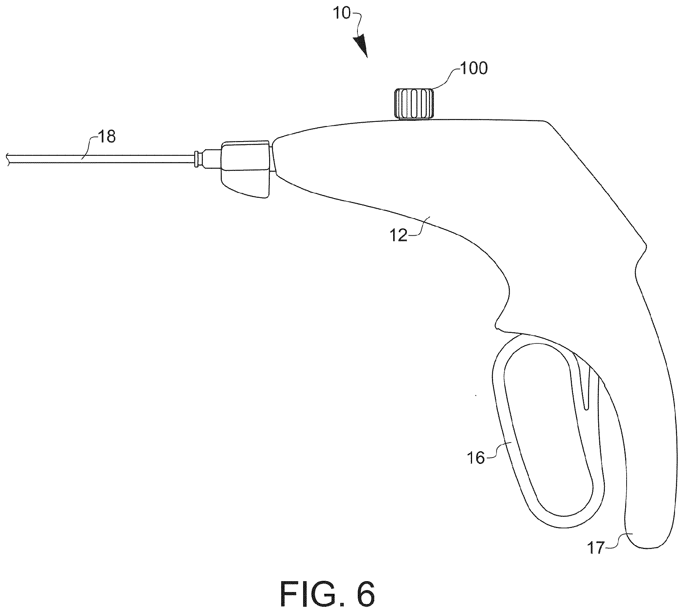

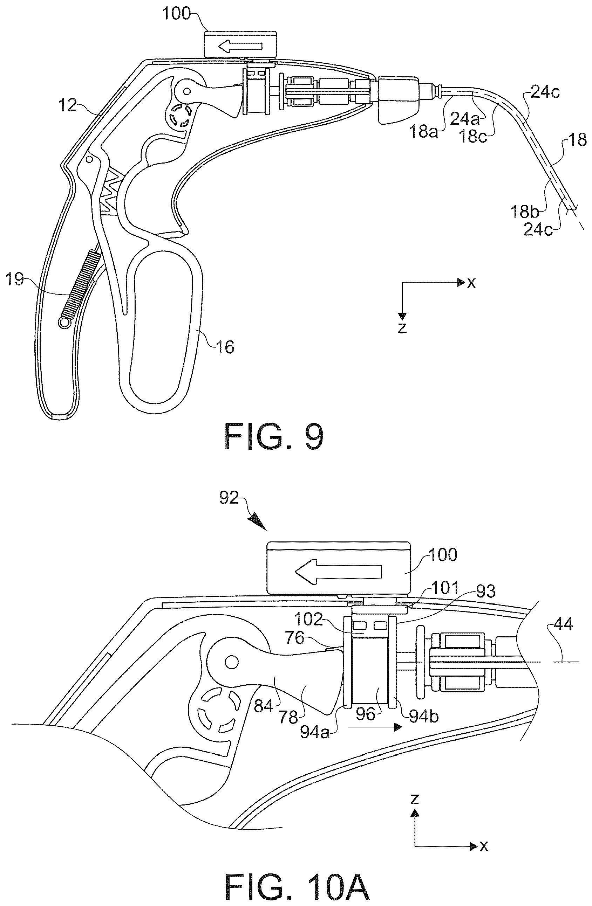

A probe device 10 includes a housing portion 12 having a grip portion 17 that is adapted to be grasped by a user to engage and displace a lever 16 that may be pivotably or rotatably coupled to a portion of the housing portion. As illustrated in Fig. 4B, the lever 16 may pivot about a pivot axis 81 that may extend parallel to the Y-axis (that is, may be normal to the X-Z plane) of the reference coordinate system of Figs. 4B and 1B). Accordingly, the lever 16 may be pivotable about the pivot axis 81 from an unactuated handle position (illustrated in Figs 1A an 4A) to an actuated handle position (illustrated in Fig. 6 and Fig 9). For example, a first portion of the lever 16 may be a pair of aligned bosses that are received into corresponding cylindrical internal walls that are each formed on a corresponding one of one or more interior surfaces at the pivot axis 81 of the housing portion 12. The one or more interior surfaces 13 may cooperate to define a housing interior 14. A portion of the lever 16 may contact a portion of the housing portion 12 when the lever 16 is in the unactuated handle position to prevent the lever 16 from overextending beyond the unactuated handle position. A first end of a spring 19 (see Fig. 9) may be coupled to a second portion of the lever 16 and a second end of the spring 19 may be coupled to a portion of the one or more interior surfaces 13 of the housing portion 12 such that the lever 16 is biased into the unactuated handle position by the spring.

As illustrated in Figs. 1A and 1B, an elongated shaft 18 may be directly or indirectly coupled to portion of the housing portion 12. The shaft 18 extends from a proximal end 20 to a distal end 22 along a shaft axis 24. The shaft 18 may be cylindrical and may have an aperture 26 that extends from the proximal end 20 to the distal end 22, and the aperture 26 may be defined by one or more interior surfaces 28 that define a shaft interior 30. In some embodiments, the one or more interior surfaces 28 may have the cross-sectional shape of a circle (when viewed along the shaft axis 24).and may define a cylindrical aperture. In some embodiments, one or more portions of the shaft 18 may be rotatably coupled to a portion of the housing portion 12 such that the shaft 18 rotates relative to a housing portion 12 about the shaft axis 24. However, in some embodiments, the shaft 18 may be fixed relative to the housing portion 12.

The shaft axis 24 may have any shape or combination of shapes. As illustrated in Fig. 1A, the shaft axis 24 may be linear from the proximal end 20 to the distal end 22. In other embodiments, one or more portions of the shaft 18 may be non-linear. For example, as illustrated in Fig. 9, an embodiment of the device 200 includes a first portion 18a of the shaft 18 that may be linear and may extend along a first portion 24a of the shaft axis 24 that may be parallel to the X-axis of the reference coordinate system of Fig. 9. A second portion 18b of the shaft 18 may be linear and extend along a second portion 24b of the shaft axis 24, and the second portion 24b may form an angle, such as an acute angle, with the X-axis of the reference coordinate system of Fig. 9. A third portion 18c of the shaft 18 may extend from a distal end of the first portion 18a to a proximal end of the second portion 18b. The second portion 18b of the shaft 18 may extend along a non-linear third portion 24c of the shaft axis 24, and the third portion 24c of the shaft axis 24 may be curved, and may have the shape of a segment of a circle when viewed along the Y-axis of the reference coordinate system of Fig. 9.

In other embodiments, two or more portions of the shaft axis 24 may be non-linear. For example, one or more portions of the shaft axis 24 may be linear, or two or more portions of the shaft axis 24 may be non-linear and two or more portions of the shaft axis 24 may be linear. The shaft 18 may be rigid, but in other embodiments, the shaft 18 may be flexible or may have one or more portions that are flexible.

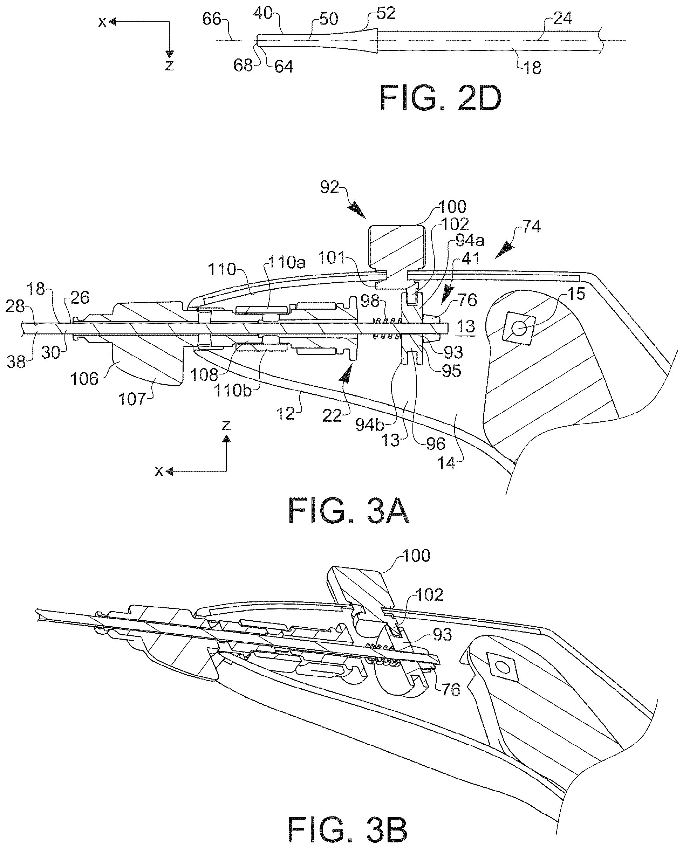

Referring to Fig. 3A, a rotation knob 106 may be coupled to the shaft 18 such that rotating the rotation knob 106 may rotate the shaft 18 about the shaft axis 24. The rotation knob 106 may be coupled to a portion of the shaft 18 that is adjacent to the proximal end of the shaft 18 and/or may be coupled to the portion of the shaft 18 that is distal to the portion of the housing portion 12 to which the shaft 18 is coupled. All or a portion of the rotation knob 32 may also be coupled to a portion of the housing portion 12. The rotational knob 106 may be configured to simultaneously rotate the shaft 18 and a probe assembly 32 that is disposed at least partially within the shaft interior 30 of the shaft 18, and the probe assembly 32 will be described in more detail below. The rotation knob 106 can have any suitable shape, and in some embodiments, may be formed as a unitary portion of the shaft 18 itself. The rotation knob 106 may also include a direction indicator 107 that may be fin or protrusion that correlates with a direction of the probe assembly 32 such that the operator of the device 10 can readily orient the housing portion 12 such that the probe assembly 32 faces a desired direction. Because it may not always be possible or ergonomically practical for a surgeon to rotate the housing portion 12 of the device, embodiments having a rotatable shaft 18 may offer more orientation flexibility to the surgeon, thereby enabling tissue and prosthetic protection.

A portion of the rotation knob 106 and/or a portion of the shaft 18 *(e.g.,* a portion of the proximal end of the shaft 18) may extend into the housing interior 13 of the housing portion 12 and the portion of the rotation knob 32 (and/or the portion of the shaft 18) may include two or more facets 108 (described and illustrated in U.S. Patent No. 10,939,904, the contents of which is incorporated by reference herein in its entirety). The facets 108 may be planar circumferential surfaces of the portion, and the facets 108 may cooperate to form a polygonal cross-sectional shape *(e.g.,* a hexagon, octagon, decagon, or dodecagon) when viewed along the shaft axis 24. In other embodiments, the facets 108 may be recesses, bumps, and/or angled edges. The facets 108 may be configured to engage corresponding engagement surfaces of a constraint assembly 110 that is disposed around the facets 108, and the engagement surfaces may correspond in cross-sectional shape with the facets 108. The constraint assembly 110 may be fixedly coupled to one or more interior portions of the housing portion 12. The constraint assembly 110 may include an upper constraint 110a and a lower constraint 110b that is coupled to the upper constraint 110a to form the constraint assembly 110. The constraint assembly 110 may be made from an injection-molded plastic material that may deform under stress.

When each facets 108 is flat against a corresponding engagement surfaces of the constraint assembly 110, rotation of the shaft 18 (to which the rotation knob 106 is coupled) within the constraint assembly 110 is resisted when a torque is applied to the shaft 18 that is below a threshold strength. However, when a sufficiently strong torque is applied to the shaft 18, the rotation of the facets 108 causes the corners between adjacent facets 108 to apply a force on the engagement surfaces of the constraint assembly 110, thereby deflecting or deforming the engagement surfaces and allowing the facets 108 and the shaft 18, to rotate relative to the engagement surfaces of the constraint assembly 110 until the next adjacent facet is flat against the corresponding engagement surface of the constraint assembly 110, thereby allowing the shaft 18 to rotate in specific increments that correspond to the angular spacing of the facets 108 about the shaft axis 24. The mating of the facets 108 with the engagement surfaces of the constraint assembly 110 may be felt by the user, thereby enabling indexing of the shaft along specific rotation positions.

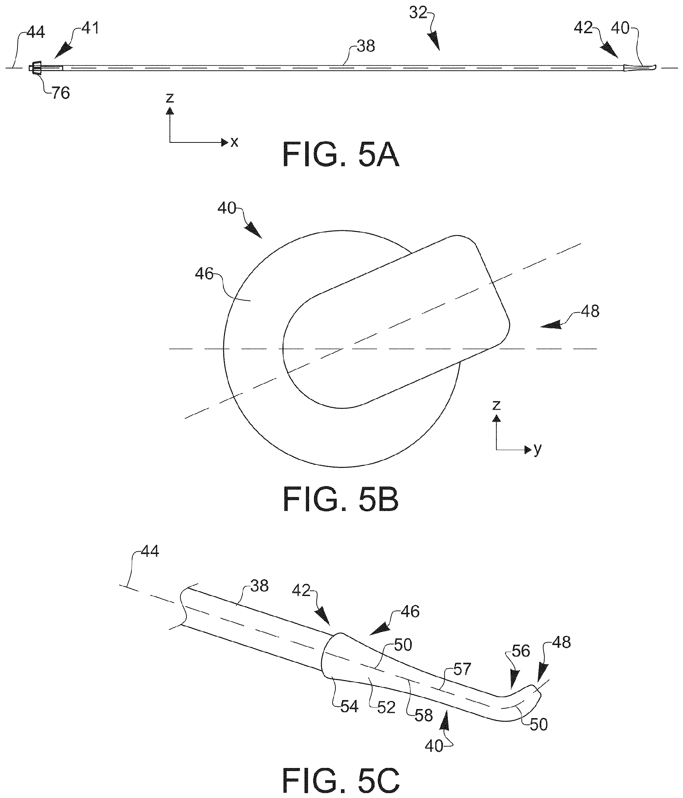

The probe device 10 includes an elongated probe assembly 32, which is illustrated in Figs. 5A to 5C. The probe assembly 32 extends from a proximal end 34 to a distal end 36, and the probe assembly 32 includes a support body 38 and a probe member 40, and the probe assembly 32 may be made from a plastic material, such as polypropylene. The support body 38 extends from a proximal end 41 to a distal end 42 along a body axis 44, and the proximal end 41 of the support body 38 is the proximal end 34 of the probe assembly 32. The probe member 40 extends from a proximal end 46 to a distal end 48 along a member axis 50, and the proximal end 46 of the probe member 40 is disposed at or adjacent to the distal end 42 of the support body 38, and the distal end 48 of the probe member 40 is the distal end 36 of the probe assembly 32.

The body axis 44 may be linear and the member axis 50 may be non-linear or partially non-linear. In some embodiments, the body axis 44 may be coaxially aligned with the shaft axis 24 such that the all or a portion of the support body 38 is disposed within the shaft interior 30. So configured, the distal end 42 of the support body 38 may be disposed at or adjacent to the distal end 22 of the shaft 18 such that the proximal end 46 of the probe member 40 is disposed exterior to the shaft interior 30. In some embodiments, the proximal end 41 of the support body 38 may be disposed proximal to the proximal end 20 of the shaft 18 such that the proximal end 41 of the support body 38 is disposed in the housing interior 14. The support body 38 may have any suitable cross-sectional shape when viewed along the body axis 44, and the cross-sectional shape may be uniform or substantially uniform along the support body 38 from the distal end 42 to the proximal end 41 or to a point that is adjacent to (or distal to) the proximal end 41. For example, the support body 38 may have a uniform circular shape (when viewed along the body axis 44), and a diameter of the circle may be slightly smaller than a corresponding diameter of the cross-sectional circle formed by the one or more interior surfaces 28 of the shaft 18. Thus, the support body 38 may be rotatable about the body axis 44 relative to the shaft 18 (within the shaft interior 30) in a manner that will be described in more detail below.

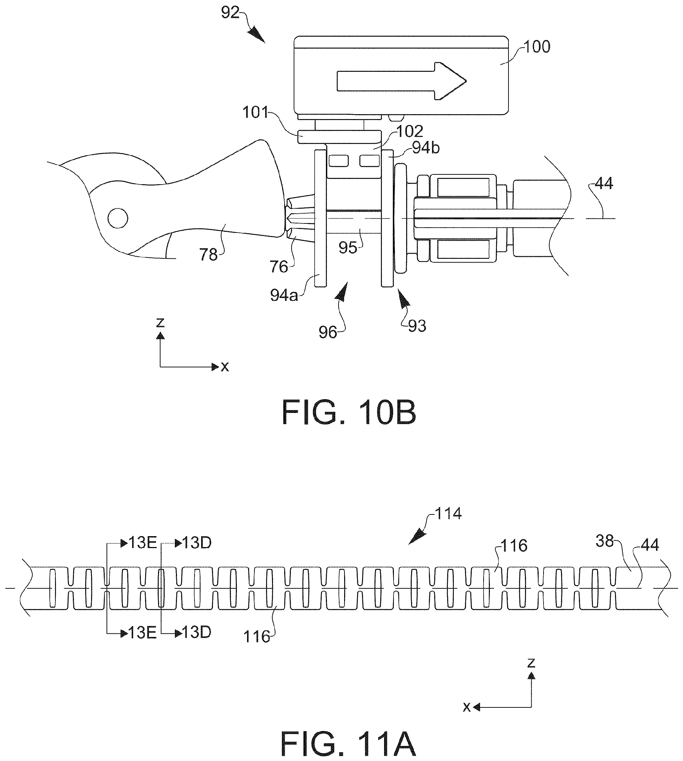

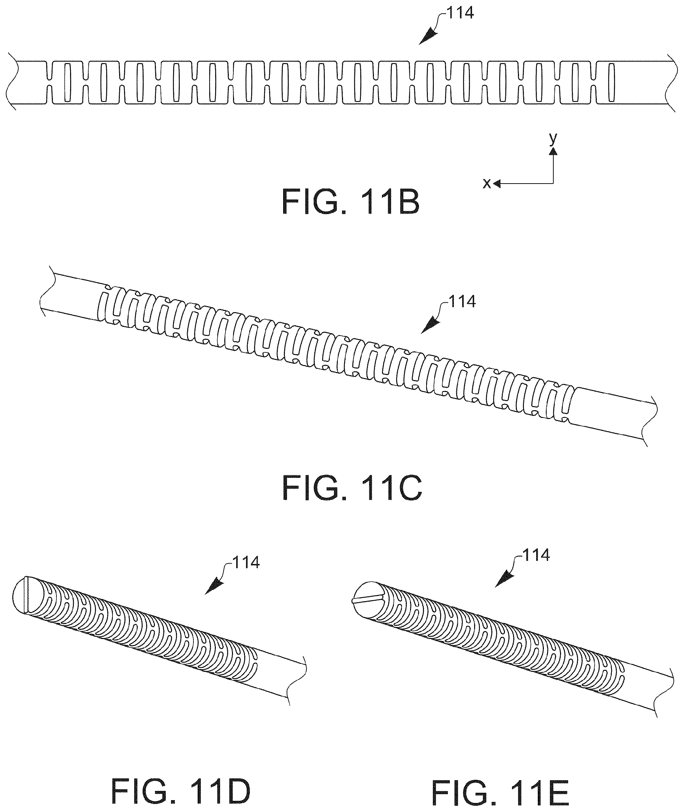

As illustrated in Fig. 9, one or more portions of the shaft 18 may be non-linear. Accordingly, one or more portions of the body axis 44 may be non-linear when all or a portion of the support body 38 is disposed within the shaft interior 30. For example, as illustrated in Figs. 11A to 11C, a portion of the support body 38 may include a hinge portion 114 that is disposed between the proximal end 41 and the distal end 42 of the support body 38 and is configured to be disposed within the third portion 18c of the shaft 18 in the embodiment of the device 200 illustrated in Fig. 11. The hinge portion 114 may have any shape or combination of shapes, or may be any feature of combination of features that allows the shaft to bend while still transmitting rotational force such that if the proximal end 41 is rotated a first angular distance in a first direction, the distal end 42 will rotate the first angular distance.

Referring to Fig. 11A, which illustrates the hinge portion 114 in a linear configuration before insertion into the shaft interior 30, the hinge portion 114 may include a plurality of disc members 116 that are offset from adjacent disc members 116, and the disc members 116 may be offset by equal distances or substantially equal distances along the hinge portion 14. Each disc member 116 may have a circular diameter that may be equal to the diameter of the support body 38. Adjacent disc members 116 have be coupled by hinge plates 118 that are disposed between adjacent disc members 116. Each of the hinge plates may be planar, and intersect that body axis 44. Each of a first plurality of hinge plates 118 extend in a plane that is parallel to the X-Z plane of the reference coordinate system of Figs 11A and 11B, and each of a second plurality of hinge plates 118 extend in a plane that is parallel to the X-Y plane of the reference coordinate system of Figs 11A and 11B. Each disc member 116 may have one of the first plurality of hinge plates 118 extending from a first surface, and one of the second plurality of hinge plates 118 extending from a second surface that is opposite to the first surface.

As illustrated in Fig 5C, the probe member 40 may extend along the member axis 50 from the proximal end 46 to the distal end 48. The probe member 40 may include a base portion 52 that extends from a proximal end 54 to a distal end 56 along a first portion 58 of the member axis 50, and the proximal end 54 of the base portion 52 may be the proximal end 46 of the probe member 40. The first portion 58 of the member axis 50 may be coaxially aligned (or substantially coaxially-aligned) with the body axis 44. The base portion 52 may have any suitable cross-sectional shape(s), and may have a circular cross-sectional shape (when viewed normal to the first portion 58 of the member axis 50). However, the diameters of the cross-sectional circles may gradually decrease from the proximal end of the base portion 52 to an intermediate point 57 proximal to the distal end 56 of the base portion 52, and the diameters of the cross-sectional circles may be equal or substantially equal from the intermediate point 57 to the distal end 56 of the base portion 52.

A tip portion 60 may extend from a proximal end 62 to a distal end 56 along a second portion 66 of the member axis 50, and the proximal end 62 of the tip portion 60 may be disposed at the distal end 56 of the base portion 52. The second portion 66 of the member axis 50 may extend along or be confined to a plane parallel to the X-Z plane of the reference coordinate system of Fig. 2D. The second portion 66 of the member axis 50 may be non-linear, and may have any suitable shape or combination of shapes. For example, the second portion 66 of the member axis 50 may have the shape of a segment of a circle and/or an arcuate shape. A distal edge 68 may be disposed at the distal end 64 of the tip portion 60, and the distal edge 68 may be linear or substantially linear, and may extend along or substantially along an axis parallel to the Y-axis of the reference coordinate system of Fig. 2D. The distal edge 68 may be an edge between a curved outer surface 70 and a curved inner surface 72, the distal edge may be radiused or rounded to avoid sharp edges that could damage tissue during a procedure. Thus, the probe member 40 may have the overall shape of an elongated hook that may be configured to atraumatically engage or contact tissue to reposition thee tissue during a procedure.

The probe device 10 may include a transmission assembly 74 that may be configured to selectively rotate the probe assembly 32 about the body axis 44, and the transmission assembly 74 may include any components that are adapted to rotate the probe assembly 32 about the body axis 44. For example, the transmission assembly 74 may include a gear 76 and a gear driver 78 to operatively engage the gear 76. The gear 76 may be any suitable gear, and may be a bevel gear. The gear 76 may be fixedly or displaceably coupled to the support body 38 proximal to the distal end 42. As illustrated in Fig. 4B, the gear 76 may be secured to or coupled to a portion of the support body 38 disposed at or adjacent to the proximal end 42 of the support body 38, and the gear 76 may be configured to displace distally along the body axis 44. In some embodiments, the gear 76 may integrally formed with a portion of the support body 38.

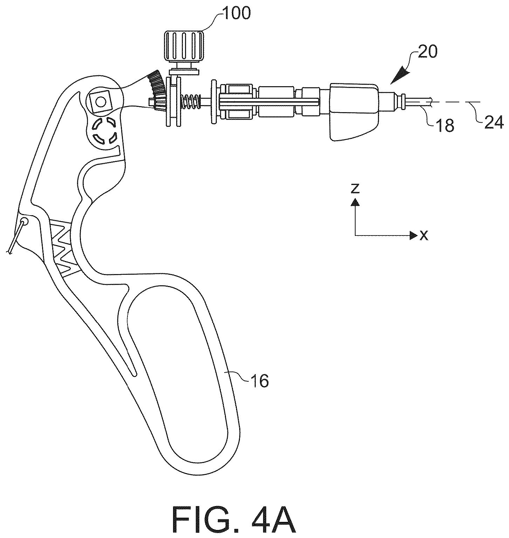

The gear driver 78 may be fixed or secured to a portion 80 of the lever 16 such that as the handle is pivoted about the pivot axis 81 from the unactuated handle position to the actuated handle position, the gear driver 78 is displaced from a first position (illustrated n Fig 4A) to a second position (illustrated in Fig 10A). The gear driver 78 may include a coupling portion 82 that may be fixed to the portion 80 of the lever 16, and the coupling portion 82 may include a projection that may be configured to be inserted into a corresponding recess or void formed in the portion 80 of the lever 16. The gear driver 78 may also include a base portion 84 that may be elongated and may extend from a proximal end to a distal end, and the coupling portion 82 may be disposed at or adjacent to the proximal end of the base portion 84. The base portion 84 may be planar or substantially planar and may have a surface that extends along the X-Z plane of the reference coordinate system of Fig 4B.

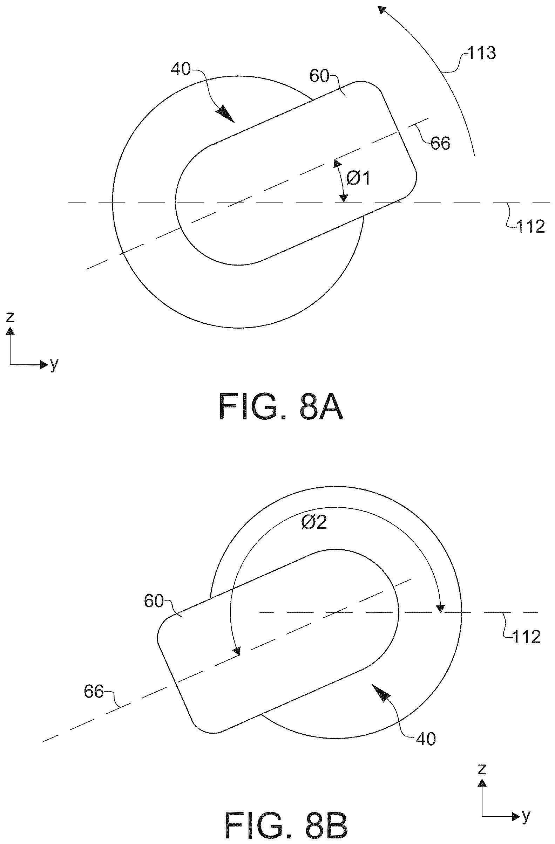

The gear driver 78 may further include an engagement portion 86 that may extend distally from the distal end of the base portion 84. The engagement portion may include any feature(s) or combination of features the rotate the gear 76 when the lever 16 is displaced from the unactuated handle position to the actuated handle position, and vice versa. For example, the engagement portion 86 may include a plurality of teeth 87 that extend from a first lateral edge 88 of the engagement portion 86 to a second lateral edge 90 of the engagement portion 86. So configured, when the lever 16 is in the unactuated handle position (illustrated in Figs. 1A and 4B), one or more of the plurality of teeth 87 that is/are adjacent to the second lateral edge 90 of the engagement portion 86 would engage one or more corresponding teeth of the bevel gear 76 in the firs position of the gear driver 76. In this position, both the bevel gear 76 and the probe member 40 are in a first position, as illustrated in Fig. 8A. In this first position, the second portion 66 of the member axis 50 that extends through the tip portion 60 of the probe member 40 (and which is viewed along the X-axis of the reference coordinate system of Figs. 8A and 8B) forms a first angle θ1 with a reference axis 112 that is parallel to the Y-axis of the reference coordinate system of Figs. 8A and 8B.

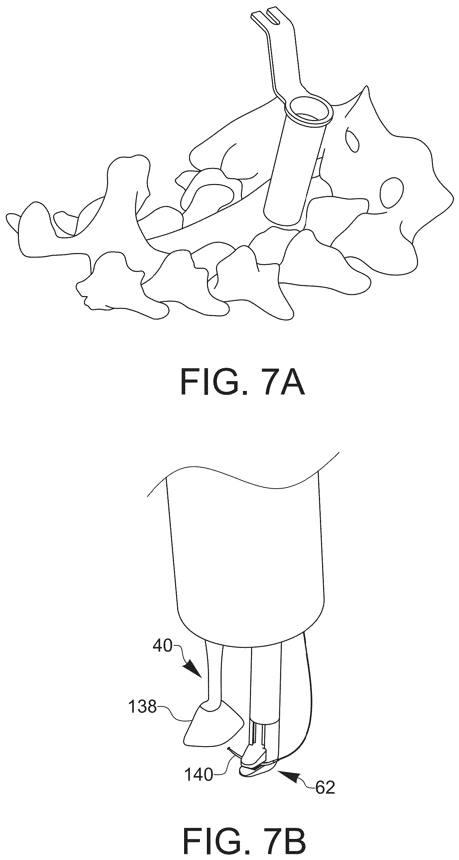

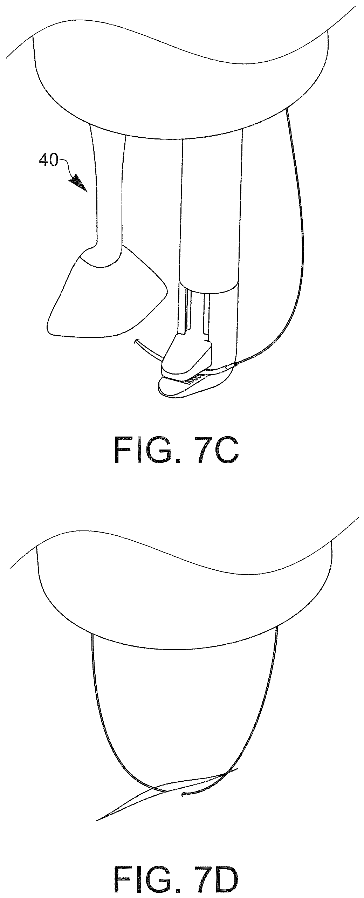

When a user grasps the lever 16 and displaces the lever 16 towards the actuated handle position (illustrated in Fig. 7), the gear driver 78 is displaced from the first position towards the second position, and the engagement portion 86 is rotated about the pivot axis 81 of the lever 16 in a plane that is parallel to the X-Z plane of the reference coordinate system of Fig. 4B. Accordingly, as the lever 16 pivots towards the actuated handle position, the one or more of the plurality of teeth 87 engage the one or more corresponding teeth of the bevel gear 76 to rotate the bevel gear 76 (and the support body 38) about the body axis 44 in a first rotational direction, thereby rotating the probe member 40 in the first rotational direction about the body axis 44, which is indicated by arrow 113 in Fig. 8A (or which may be opposite to the direction which is indicated by arrow 113 in Fig. 8A). Thus, the bevel gear 76 and the probe member 40 are rotated from the first position (illustrated in Fig. 8A) towards a second position (illustrated in Fig. 8B). As the lever 16 is rotated to the actuated handle position, the gear driver 78 is displaced into the second position. Thus, the one or more of the plurality of teeth 87 is/are adjacent to the first lateral edge 98 of the engagement portion 86, and the bevel gear 76 and probe member 40 are rotated into the second position. As illustrated in Fig. 8B, in this second position, the second portion 66 of the member axis 50 that extends through the tip portion 60 of the probe member 40 (and which is viewed along the X-axis of the reference coordinate system of Figs. 8A and 8B) forms a second angle θ2 with a reference axis 112 that is parallel to the Y-axis of the reference coordinate system of Figs. 8A and 8B. Thus, the total rotational sweep of the probe member 40 from the first position to the second position may be equal to the first angle θ1 subtracted from the second angle θ2. The total rotational sweep may be any suitable angle value, such as between 30° and 270°, between 130° and 200°, or between 140° and 190°, or between 160° and 190°.

When the user releases the lever 16, the lever 16 is biased into pivoting to the unactuated handle position by the spring, and the actuation process is reversed, thereby rotating the bevel gear 76 and the probe member 40 from the second position to the first position. Such rotation allows a user to manipulate tissue during a procedure to position a first portion of the tissue in a first position for a first portion of suturing, and the user may rotate only the probe member 40 (and not the housing portion 12) to position a second portion of the tissue in a second position for a second portion of suturing, as illustrated in Figs. 7A to 7D.

The transmission assembly 74 may also include a clutch assembly 92 that may be configured to disengage the gear 76 from the gear driver 78 to reposition the probe member 40 to a suitable initial position and then reengage the gear 76 with gear driver 78 to allow the probe member 40 to be actuated as described. In some embodiments, the gear 76 may be displaced distally along the body axis 44 by the clutch assembly 92 to disengage the gear 76 for repositioning the probe member 40 (as illustrated in Fig 10B), and the gear 76 may be displaced proximally along the body axis 44 by the clutch assembly 92 to reengage the gear 76 after a desired initial position of the probe member 40 has been determined.

For example, as illustrated into the cross-sectional view of Fig. 3A, the clutch assembly 92 may include a disc assembly 93 that may include a first clutch disc 94a that may be disposed around a portion of the support body 38 (or coupled to a portion of the support body 38), and the gear 76 may be fixedly coupled to a proximal portion of the first clutch disc 94a. The disc assembly 93 may include a second clutch disc 94b may be distally offset from the first clutch disc 94a. The disc assembly 93 may also include a hub 95 that couples the first clutch disc 94a and the second clutch disc 94b, and a circumferential groove 96 may be defined between a distal surface of the first clutch disc 94a and a proximal surface of the second clutch disc 94b. A central axis of the first and second clutch discs 94a, 94b may be coaxially aligned with the body axis 44, and the disc assembly 93 may be coupled to the support body 38 such that the first and disc assembly 93 may rotate along the with the support body 38 as the support body 38 rotates about the body axis 44. However, the disc assembly 93 may be configured to displace along the body axis 44, and the disc assembly 93 may be biased in a first direction (e.g., proximally along the X-axis of the reference coordinate system of Fig. 3A), and the gear 76 may be biased into engagement with the gear driver 78, by a spring 98.

The clutch assembly 92 may further include a clutch knob 100 that may be rotatably coupled to a portion of the housing portion 12, and a plate 101 may be coupled to a portion of the clutch knob 100, and the plate 101 may be disposed in the housing interior 14. The plate 101 may be fixedly coupled with the portion of the clutch knob 100, and the plate 101 may be coaxially aligned with a central axis of the clutch knob 100, and the central axis may be parallel to the Z-axis of the reference coordinate system of Fig. 3A. Accordingly, a rotation of the clutch knob 100 about the central axis results in a corresponding rotation of the plate 101 about the central axis. A projection 102 extends from a bottom surface of the plate 101, and the projection 102 extends along an axis that may be parallel to and offset from the central axis such that the projection 102 is disposed within a portion of the circumferential groove 96 of the disc assembly 93. The projection 102 may have the shape of a cylinder and may have a diameter that is equal or substantially equal to the length (along the X-axis) of the circumferential groove 96.

So configured, starting from the engaged position of Fig. 10A, a rotation of the clutch knob 100 about the central axis will displace the projection 102 along the X-axis of the reference coordinate system. Thus, when the clutch knob 100 is rotated in a first direction towards the disengaged position of Fig. 10B, the projection 102 moves distally along the X-axis of the reference coordinate system of Fig. 3A, and the projections 102, which is disposed in the circumferential groove 96, displaces the disc assembly 93 (and the gear 76 secured to or formed with the first clutch disc 94a) distally, thereby disengaging the gear 76 from the gear driver 78, as shown in the fully disengaged position illustrated in Fig. 10B, with the clutch knob 100 rotated 180° from the initial position illustrated in Fig. 10A. Thus, with the clutch assembly 92 in the disengaged position of Fig. 10B, the probe member 40 may be rotated about the shaft axis 24 (or the member axis 44) to find a suitable initial position for the probe member 40 relative to the distal end 22 of the shaft 18. With the desired initial position located, the knob member 100 may be rotated such that that the clutch assembly 92 in the engaged position of Fig. 10A and may be ready for use. If a different initial position of the probe member 40 is subsequently desired, the clutch knob 100 may be rotated back into the disengaged position of Fig. 10B and the process may be repeated.

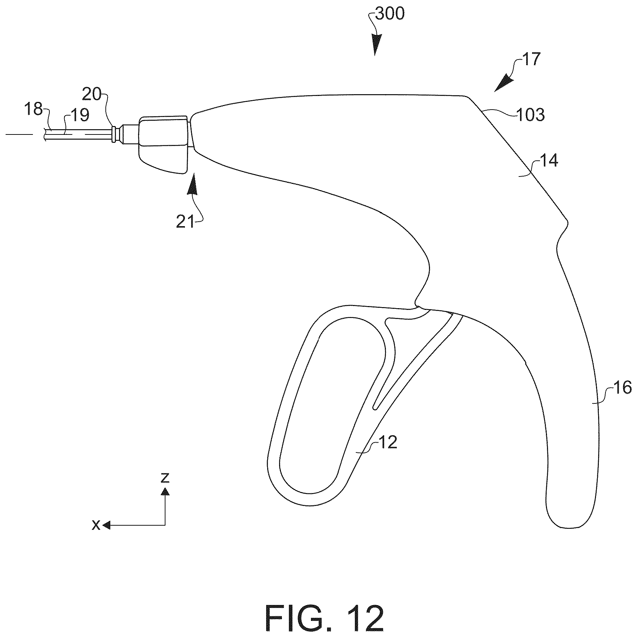

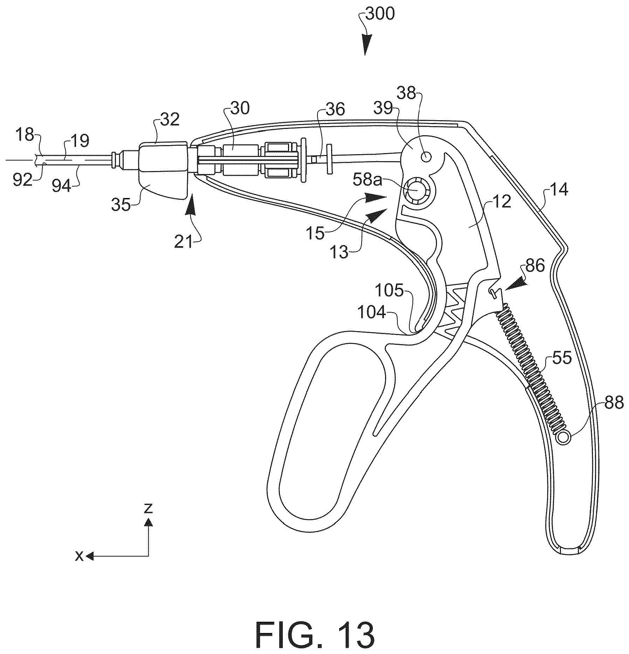

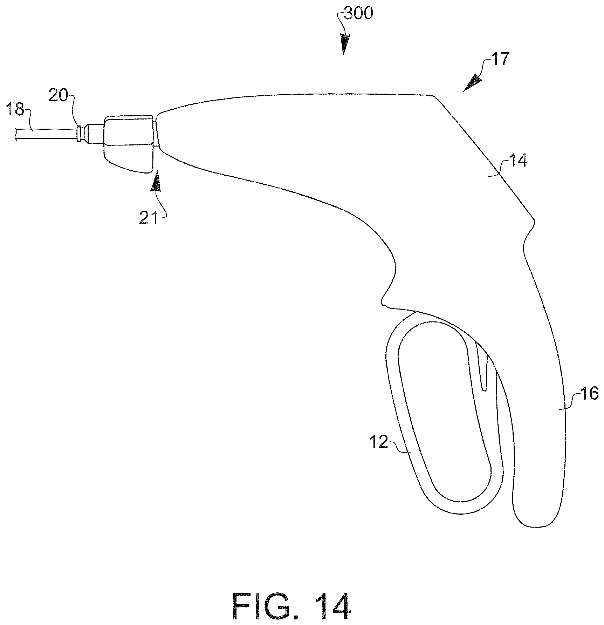

A further device 300, which may be a grasping device, is illustrated in Figs. 14-22. The device 300 includes a housing portion 14 having a grip portion 16 that is adapted to be grasped by a user to engage and displace a lever 12 from a first lever position (illustrated in Figure 12) to a second lever position (illustrated in Figure 14). As illustrated in Fig. 13 (in which a portion of the housing portion 14 is omitted for clarity), a first portion of the lever 12 may be rotatably coupled to the housing portion 14 at a first portion 15 *(e.g.,* a pivot point) of the housing portion 14 such that the lever 12 pivots between the first lever position to the second lever position about a pivot axis that extends through the pivot point 15, and the pivot axis may be parallel to the Y-axis of the reference coordinate system of Fig. 12. The pivot may be normal to a shaft axis 19 that extends along the shaft 18 that is coupled to the housing portion 14. In particular, the first portion of the lever 12 may be a pair of aligned bosses that are received into corresponding cylindrical internal walls that are each formed on a corresponding interior portion of the housing portion 14 at the pivot point 15 of the housing portion 14. A portion of the lever 12 may contact a portion of the housing portion 14 when the lever 12 is in the first lever position to prevent the lever 12 from overextending beyond the first lever position. A first end of a spring 55 may be coupled to a second portion 86 of the lever 12 and a second end of the spring 55 may be coupled to a portion 88 of the interior portion of the housing portion 14 such that the lever 12 is biased into the first lever position by the spring 55. The lever may be selectively locked by a locking assembly 120 that may be identical to that described in U.S. Patent Application No. 18/107,392, entitled "DEVICE FOR VESSEL HARVESTING" and filed on February 8, 2023 , the contents of which is incorporated by reference herein in its entirety.

Still referring to Figure 13, the device 300 includes the shaft 18 that extends from a proximal end 20 to a distal end 22 along the shaft axis 19, and one or more portions of the shaft 18 may be coupled to a second portion 21 of the housing portion 14, and the shaft 18 may be fixed relative to the housing portion 12. However, in some embodiments, one or more portions of the shaft 18 may be rotatably coupled to the second portion 21 of the housing portion 14 such that the shaft 18 rotates relative to a housing portion 14 about the shaft axis 19.

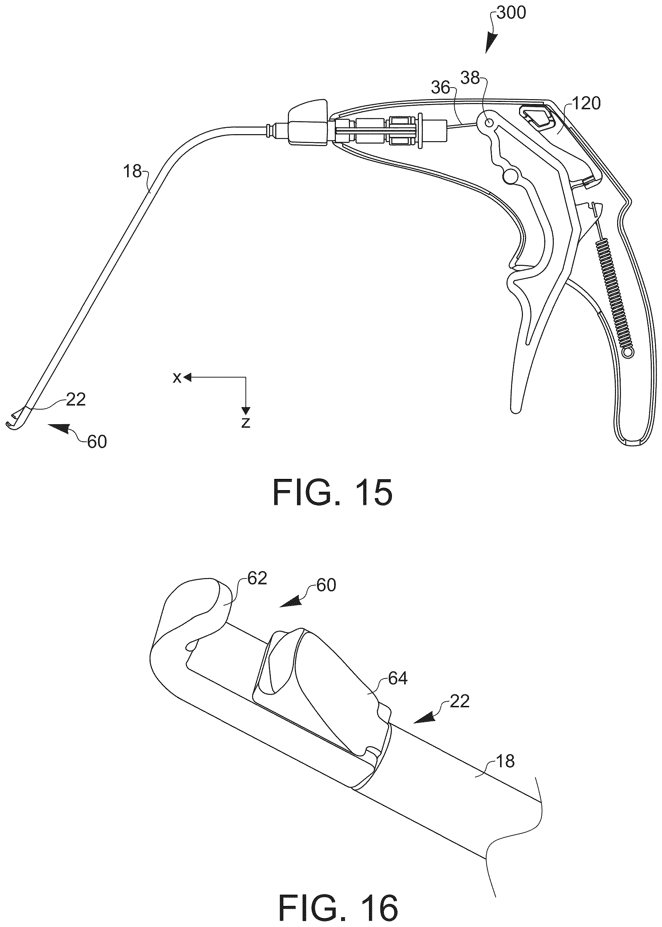

The shaft axis 19 may have any shape or combination of shapes. In some embodiments, the shaft axis 24 may be linear from the proximal end 20 to the distal end 22. In other embodiments, one or more portions of the shaft 18 may be non-linear. For example, as illustrated in Fig. 15, an embodiment of the device 300 includes a first portion 18a of the shaft 18 that may be linear and may extend along a first portion 19a of the shaft axis 19 that may be parallel to the X-axis of the reference coordinate system of Fig. 15. A second portion 18b of the shaft 18 may be linear and extend along a second portion 19b of the shaft axis 19, and the second portion 18b may form an angle, such as an acute angle, with the X-axis of the reference coordinate system of Fig. 15. A third portion 18c of the shaft 18 may extend from a distal end of the first portion 18a to a proximal end of the second portion 18b. The third portion 18c of the shaft 18 may extend along a non-linear third portion 19c of the shaft axis 19, and the third portion 19c of the shaft axis 19 may be curved, and may have the shape of a segment of a circle when viewed along the Y-axis of the reference coordinate system of Fig. 15. The shaft 18 may be rigid, but in other embodiments, the shaft 18 may be flexible or may have one or more portions that are flexible.

The shaft 18, or one or more portions of the shaft 18, may have the general shape of an elongated hollow tube having one or more interior surfaces that defines an interior portion 94 extends from the proximal end 20 to the distal end 22 of the shaft 18. The shaft 18 and the interior surface 92 may have any suitable cross-sectional shape or combination of shapes normal to the shaft axis 19. For example, the shaft 18 may have the general shape of an elongated cylinder, and the interior surface 92 may have a circular cross-sectional shape when viewed normal to the shaft axis 19.

A rotation knob 32 may be coupled to the shaft 18 such that rotating the rotation knob 32 may rotate the shaft 18 about the shaft axis 19. The rotation knob 32 may be coupled to a portion of the shaft 18 that is adjacent to the proximal end 20 of the shaft 18 and/or may be coupled to the portion of the shaft 18 that is distal to the portion of the housing portion 12 to which the shaft 18 is coupled. All or a portion of the rotation knob 32 may also be coupled to a portion of the housing portion 14, such as the second portion 21 of the housing portion 14. The rotational knob 32 may be configured to simultaneously rotate the shaft 18 and a push rod 36 that is disposed at least partially within the interior portion 94 of the shaft 18. The rotation knob 32 can have any suitable shape, and in some embodiments, may be formed as a unitary portion of the shaft 18 itself. The rotation knob 32 may also include a direction indicator 33 that may be fin or protrusion that correlates with a direction of the needle assembly 24 such that the operator of the device 300 can readily orient the housing portion 14 such that a jaw assembly 60 faces a desired direction. Because it may not always be possible or ergonomically practical for a surgeon to rotate the housing portion 14 of the device, embodiments having a rotatable shaft 18 may offer more orientation flexibility to the surgeon, thereby enabling tissue and prosthetic protection.

With reference to Figure 13, a portion 96 of the rotation knob 32 and/or a portion of the shaft 18 (*e.g.,* a portion of the proximal end 20 of the shaft 18) may extend into the interior of the housing portion 14 and the portion 96 of the rotation knob 32 (and/or the portion of the shaft 18) may include two or more facets 98 (described and illustrated in U.S. Patent No. 10,939,904, the contents of which is incorporated by reference herein in its entirety). The facets may be planar circumferential surfaces of the portion 96, and the facets 98 may cooperate to form a polygonal cross-sectional shape (*e.g.,* a hexagon, octagon, decagon, or dodecagon) when viewed along the shaft axis 19. In other embodiments, the facets 98 may be recesses, bumps, and/or angled edges. The facets 98 may be configured to engage corresponding engagement surfaces of a constraint assembly 30 that is disposed around the facets 98, and the engagement surfaces may correspond in cross-sectional shape with the facets 98. The constraint assembly 30 may be fixedly coupled to one or more interior portions of the housing portion 14. The constraint assembly 30 may include an upper constraint 30a and a lower constraint 30b that is coupled to the upper constraint 30a to form the constraint assembly 30. The constraint assembly 30 may be made from an injection-molded plastic material that may deform under stress.

When each facet 98 is flat against a corresponding engagement surfaces of the constraint assembly 30, rotation of the shaft 18 (to which the rotation knob 32 is coupled) within the constraint assembly 30 is resisted when a torque is applied to the shaft 18 that is below a threshold strength. However, when a sufficiently strong torque is applied to the shaft 18, the rotation of the facets 98 causes the corners between adjacent facets 98 to apply a force on the engagement surfaces of the constraint assembly 30, thereby deflecting or deforming the engagement surfaces and allowing the facets 98 and the shaft 18, to rotate relative to the engagement surfaces of the constraint assembly 30 until the next adjacent facet is flat against the corresponding engagement surface of the constraint assembly 30, thereby allowing the shaft 18 to rotate in specific increments that correspond to the angular spacing of the facets 98 about the shaft axis 19. The mating of the facets 98 with the engagement surfaces of the constraint assembly 30 may be felt by the user, thereby enabling indexing of the shaft along specific rotation positions.

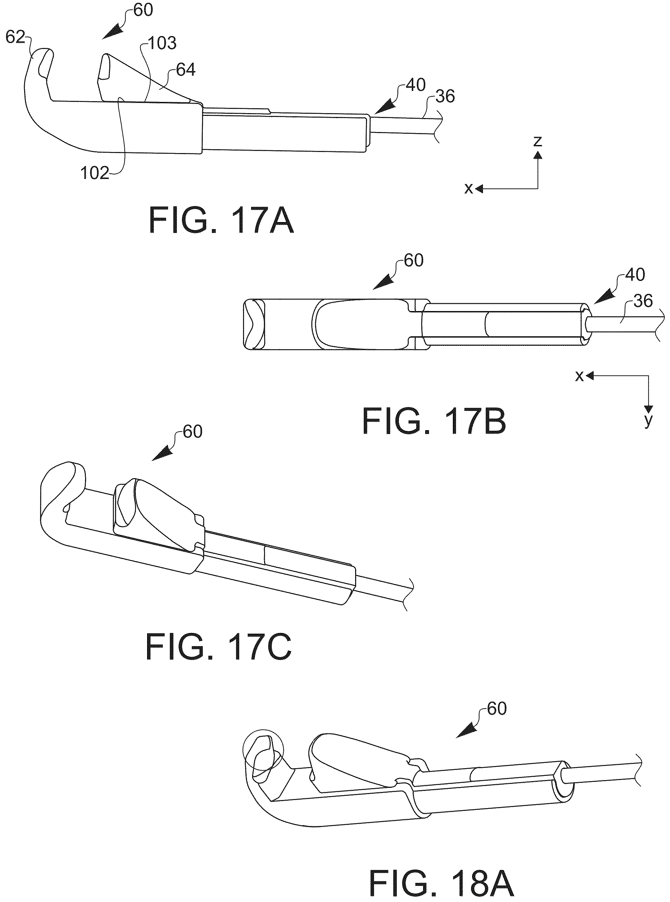

Still referring to Figure 13, the device 300 also includes a push rod 36 extending along a push rod axis 37 from a proximal end 38 to a distal end 40 (illustrated in Fig 17A, in which the shaft 18 is omitted for clarity). The push rod 36 is elongated and at least a portion of the push rod 36 extends through the interior portion 94 of the shaft 18. The proximal end 38 of the push rod 36 is coupled to a third portion 39 of the lever 12 such that when the lever 12 is in the first lever position, the push rod 36 is in a first rod position, as illustrated in Figure 13. When the lever 12 is pivoted to the second lever position, (illustrated in Figures 14) the push rod 36 is in a second rod position in which the distal end 40 of the push rod 36 (and the entire push rod 36) is displaced distally from the first position. The push rod 36 may translate linearly or substantially linearly from the first rod position to the second rod position along (or substantially along) the push rod axis 37, and the push rod axis 37 may extend parallel to (or substantially parallel to) or along the shaft axis 19.

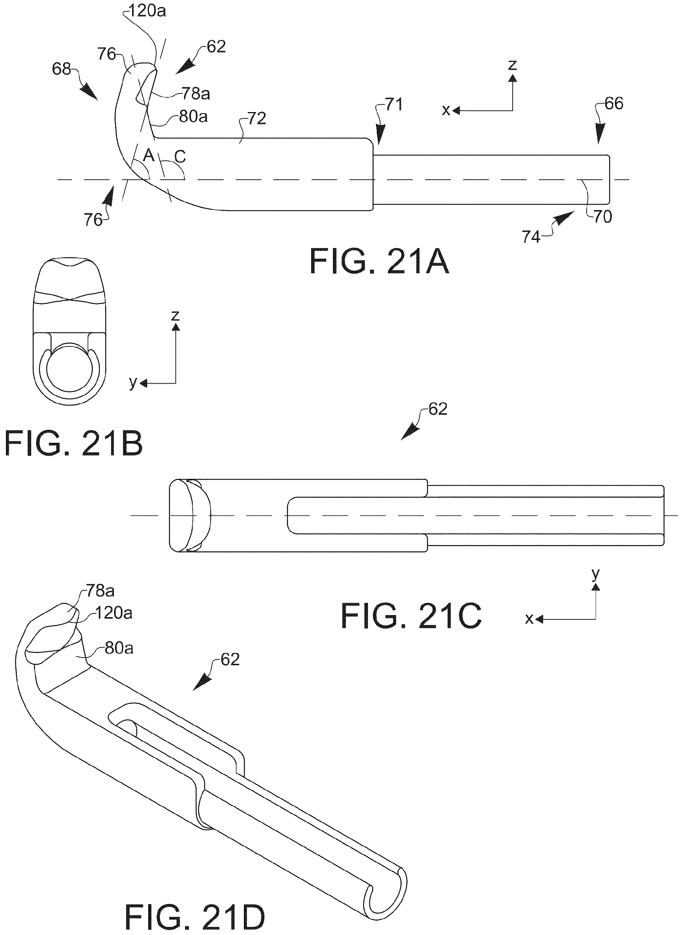

With reference to Fig. 15, the device 300 may also include a jaw assembly 60 disposed at the distal end 22 of the shaft 18. As illustrated in Fig. 16, the jaw assembly 60 may include a first jaw 62 that may be fixedly coupled to the distal end 22 of the shaft 18 and a second jaw 64 that may be displaceably coupled to the distal end 22 of the shaft 18. As illustrated in Figs. 21A to 21D, the first jaw 62 may extend from a proximal end 66 to a distal end 68 along a first jaw axis 70, and the proximal end 68 may be disposed within the interior portion 94 of the shaft 18 such that the proximal end 68 is proximal to the distal end 22 of the shaft 18. An intermediate point 71 may be disposed between the proximal end 66 and the distal end 68, and the intermediate point may be disposed at or adjacent to the distal end 22 of the shaft 18. The first jaw 62 may include a first jaw body 72 that may extend from a proximal end 74 to a distal end 76 along the first jaw axis 70, and the proximal end 74 and distal end 76 of the first jaw body 72 may be disposed at the proximal end 66 and the distal end 68, respectively, of the first jaw 62. A channel 65 may extend along a portion of the first jaw body 72 along the first jaw axis 70 from a proximal end to a distal end. The proximal end of the channel 65 may be disposed at the proximal end 74 of the first jaw body 72, and the distal end of the channel 65 may be disposed between the intermediate point 71 and the distal end 76 of the first jaw body 72.

The first jaw 62 may also include a first jaw projection 76 that may extend from the distal end 76 of the first jaw body 72 along an axis that is normal or substantially normal to the first jaw axis 70, and the first jaw projection 76 may extends along or substantially along the Z-axis of the reference coordinate system of Fig. 21A. The first jaw projection 76 may be defined by a plurality of surfaces, and the plurality of surfaces may include a proximally-facing first upper engagement surface 78a and a first lower engagement surface 80a that may be defined in more detail below.

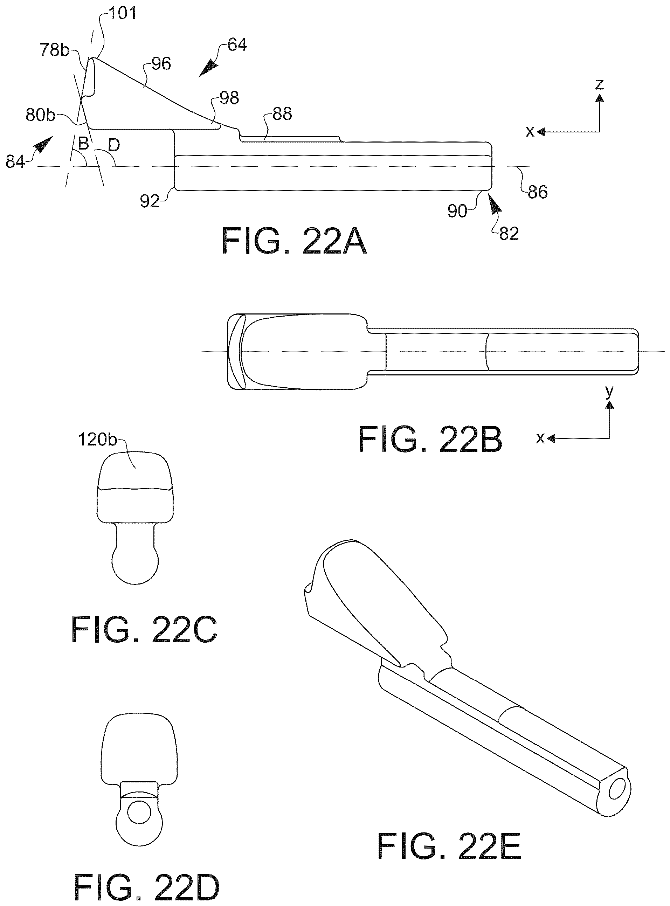

As illustrated in Fig. 16, the second jaw 64 that may be displaceably coupled to the distal end 22 of the shaft 18 such that the second jaw 64 displaces relative to the first jaw 64 along the first jaw axis 70. As illustrated in Figs. 22A to 22E, the second jaw 64 may extend from a proximal end 82 to a distal end 84 along a second jaw axis 86, and the proximal end 82 may be disposed within the interior portion 94 of the shaft 18 such that the proximal end 68 is proximal to the distal end 22 of the shaft 18. The second jaw 64 may include a second jaw body 88 that may extend from a proximal end 90 to a distal end 92 along the second jaw axis 86, and the proximal end 90 of the second jaw body 88 may be disposed at the proximal end 82 of the second jaw 64. The distal end 40 of the push rod 36 may be coupled to the proximal end 90 of the second jaw body 88. All or a portion of the second jaw body 88 may be configured to be slidably disposed within all of a portion of the channel 65 of the first jaw body 72 such that when the push rod 36 is displaced relative to the first jaw 62, the second jaw 64 displaced relative to the first jaw 62 along the first jaw axis70 (of the second jaw axis 86).

The second jaw 64 may also include a second jaw projection 96 that may extend from a portion of the second jaw body 72 that may be at or adjacent to the distal end 92 of the second jaw body 88. In particular, the second jaw projection 96 may extend from a proximal end 98 to a distal end 101, and the proximal end 98 may be proximal to the distal end 92 of the of the second jaw body 72, while the distal end 101 may extend distally beyond the distal end 92 of the second jaw body 72. The second jaw projection 96 may extend from a top, planar surface of the second jaw body 72, and the second jaw projection 96 may extend may have a substantially triangular shape when viewed along the Y-axis of the reference coordinate system of Figs. 22A and 22B. When the portion of the second jaw body 88 is disposed within all of a portion of the channel 65 of the first jaw body 72, a bottom lateral portion 102 of the second jaw projection 96 is disposed adjacent to a top lateral edge portion 103 of the channel 65 of the first jaw body 72, as illustrated in Fig. 17A.

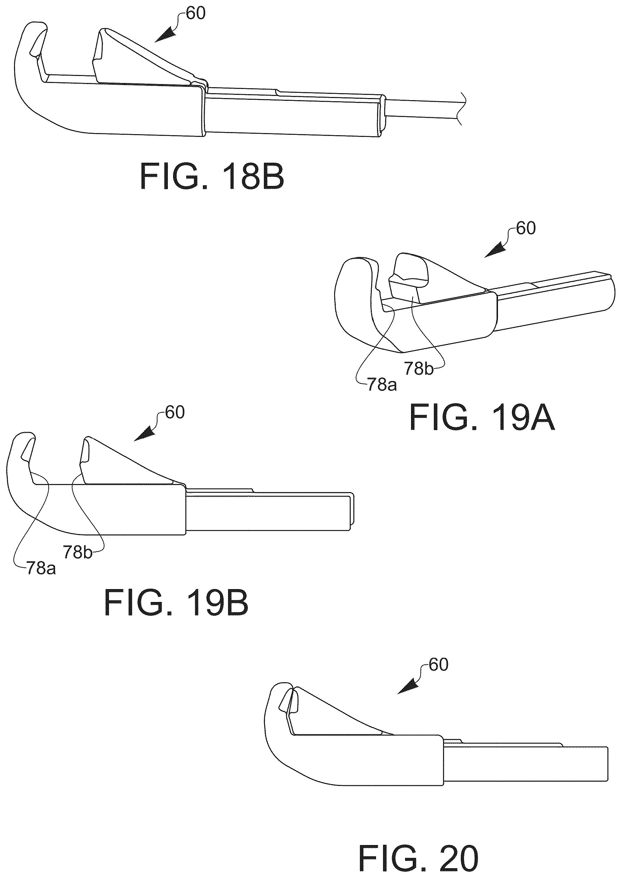

The second jaw projection 96 may be defined by a plurality of surfaces, and the plurality of surfaces may include a distally facing second upper engagement surface 78b and a second lower engagement surface 80b. When the jaw assembly 62 is in an engaged position (illustrated in Fig. 20) in which the second jaw 64 is displaced distally (.e.g., by displacing or pivoting the lever 12 towards the grip portion 16, as illustrated in Fig. 14) which displaces the push rod 36 distally), all or a portion of the second upper engagement surface 78b of the second jaw projection 96 may be brought adjacent to or into engagement with all or a portion of the first upper engagement surface 78a of the first jaw projection 76, and all or a portion of the second lower engagement surface 80b of the second jaw projection 96 may be brought adjacent to or into engagement with all or a portion of the first lower engagement surface 80b of the first jaw projection 76.

The first lower engagement surface 80a of the first jaw projection 76 may be planar but may form an acute angle with the Z-Y plane when viewed along an axis parallel to the Y-axis, as illustrated in Fig. 21A, such that the plane forms an angle A (such as an angle between 95°to 135°) with the axis 70 (which is parallel to the X-axis) when viewed along an axis parallel to the Y-axis, as illustrated in Fig. 21A. Similarly, the second lower engagement surface 80b of the second jaw projection 96 may be planar but may form an acute angle with the Z-Y plane when viewed along an axis parallel to the Y-axis, as illustrated in Fig. 21A, such that the plane forms an angle B (such as an angle between 95°to 135°) with the axis 86 (which is parallel to the X-axis) when viewed along an axis parallel to the Y-axis, as illustrated in Fig. 22A. The angles A and B may be any suitable value, and in some embodiments, may be identical or substantially identical (or complementary), such as an angle between 95°to 105°, as illustrated in Figs. 19A, 19B, and 20, such that the surfaces 80a, 80b are parallel when viewed along an axis parallel to the Y-axis.

Thus, both the second lower engagement surface 80b of the second jaw projection 96 and the first lower engagement surface 80b of the first jaw projection 76 slant distally as each of the surfaces extends away from the first jaw axis 70. Thus, these surfaces 80a, 80b cooperate in the engaged position to maximize surface area when gripping a needle during a procedure to allow for more purchase with gripping the needle. Further, the slanted feature on the flat surfaces bias the needle down during the "driving" step of taking a bite. Thus, the slanted feature of the surfaces provides increased control of the orientation of the needle when driving it through tissue.

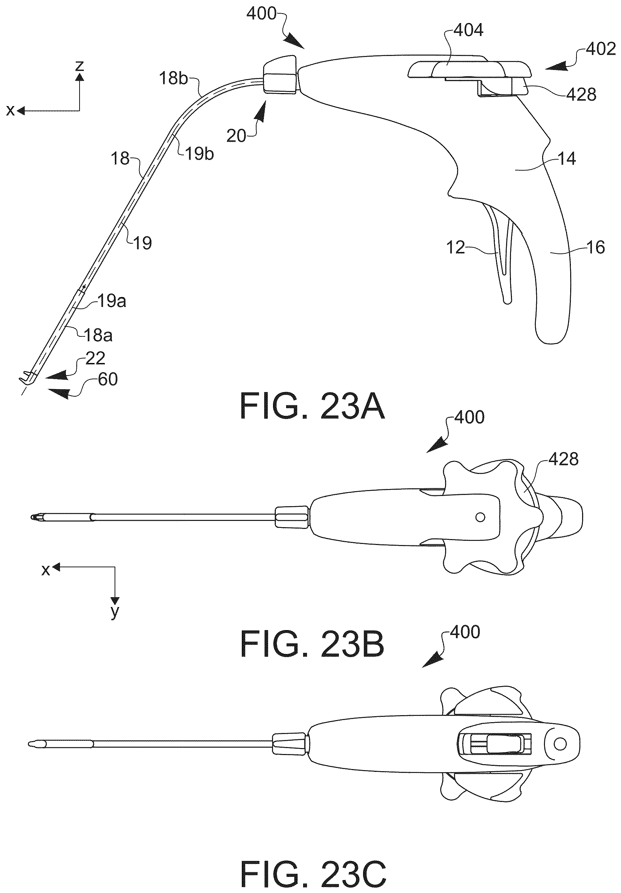

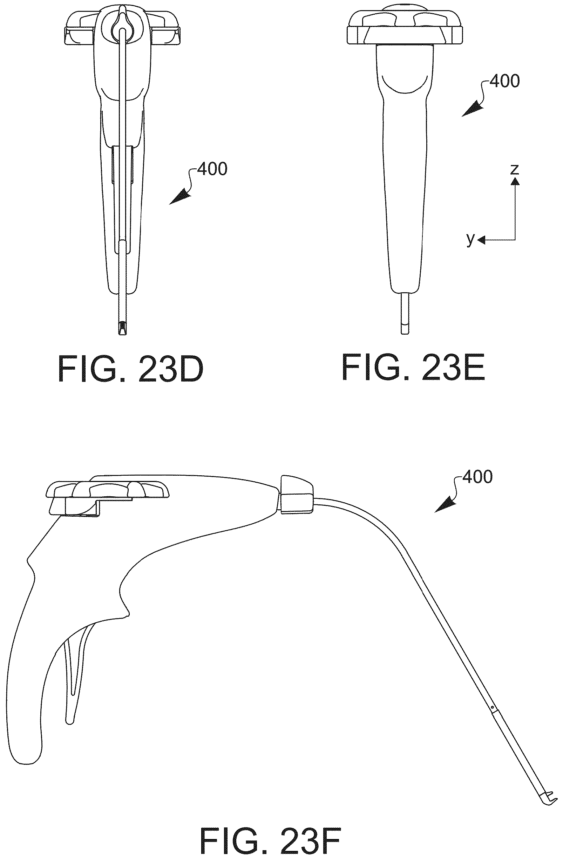

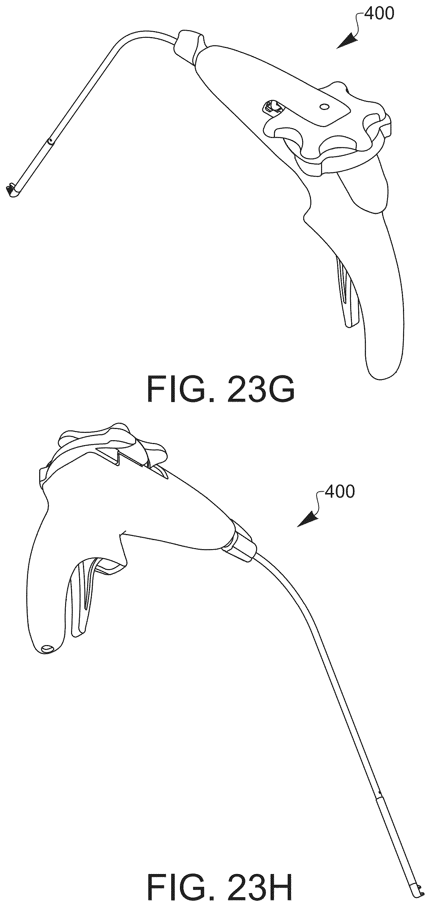

The first upper engagement surface 78a of the first jaw projection 76 may be planar but may form an acute angle with the Z-Y plane when viewed along an axis parallel to the Y-axis, as illustrated in Fig. 21A, such that the plane forms an angle C (such as an angle between 55°to 85°) with the axis 70 (which is parallel to the X-axis) when viewed along an axis parallel to the Y-axis, as illustrated in Fig. 21A. The first upper engagement surface 78a may include an upper protrusion 120a having an apex that defines an edge that is parallel to the slope of the first upper engagement surface 78a when viewed along an axis parallel to the y-axis, as illustrated in Fig 23A. Similarly, the second upper engagement surface 78b of the second jaw projection 96 may be planar but may form an acute angle with the Z-Y plane when viewed along an axis parallel to the Y-axis, as illustrated in Fig. 22A, such that the plane forms an angle D (such as an angle between 55°to 85°) with the axis 86 (which is parallel to the X-axis) when viewed along an axis parallel to the Y-axis, as illustrated in Fig. 22A. The second upper engagement surface 78b may include an upper protrusion 120b having an apex that defines an edge that is parallel to the slope of the second upper engagement surface 78b when viewed along an axis parallel to the y-axis, as illustrated in Fig 23A. The angles C and D may be any suitable value, and in some embodiments, may close by not identical, and each may have an angle between 75°to 85°, as illustrated in Figs. 19A, 19B, and 20, such that the surfaces 78a, 78b (and the apex edges of the upper protrusions 120a, 120b) are not parallel when viewed along an axis parallel to the Y-axis, as viewed in Fig. 20. Thus, when the jaw assembly 60 is in the engaged position of Fig. 20, the apex edges of the upper protrusions 120a, 120b converge and may have a single point of contact to grasp a needle between the apex edges of the upper protrusions 120a, 120b.

The first and second upper engagement surfaces 78a, 78b are used during the retrieval of the needle after pushing it through tissue to pull it out and get the desired tension on the suture. The upper protrusions 120a, 120b prevents biasing the orientation of the needle when the surgeon grabs it to pull it out.

The apex edges of the upper protrusions 120a, 120b converge to bias the needle down (into the biting regions of the jaws). If the jaws were parallel or slanted in the opposite direction, you risk pushing the needle out when gripping it. (see last image with side view of jaws).

In a procedure, as illustrated in Figs. 7A, a surgeon may access a portion of a spine and insert a cannula to access the area. The low-profile probe member 40 of the probe device 10 may be inserted into the cannula to manipulate tissue surrounding an incision 138 in the dura to allow a surgeon to suture the incision by grasping a needle 140 with the jaw assembly 60 of the device 300, which is also inserted in the cannula. The shafts 18 of each of the devices are bent so that the surgeon has unimpeded visual access to the treatment area. When one side of the dura is sutured (as illustrated in Fig. 7D), the probe member 40 can be easily rotated by the surgeon as described without removal from the cannula to manipulate the opposite side of the tissue surrounding the incision 138 to allow the incision to be fully sutured.

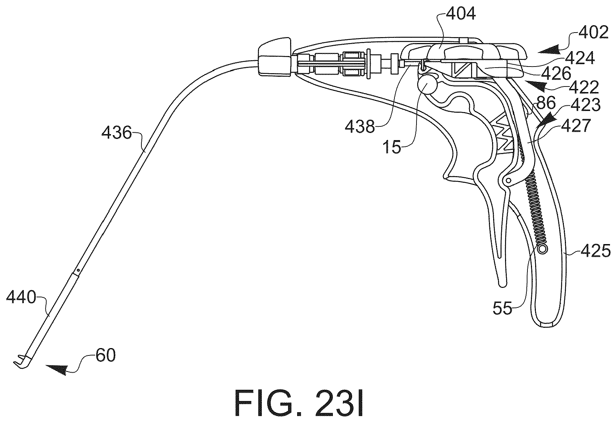

A further device 400 is illustrated in Figs. 23A to 23H. The device 400 is similar to the device 300, and features of the device 400 that are similar or identical to features of the device 300 will use like reference numbers. In particular, the device 400 includes a housing portion 14 having a grip portion 16 that is adapted to be grasped by a user to engage and displace a lever 12 from a first lever position (illustrated by device 300 in Figure 12) to a second lever position (illustrated in Figure 23A). As illustrated in Fig. 23I (in which a portion of the housing portion 14 is omitted for clarity), a first portion of the lever 12 may be rotatably coupled to the housing portion 14 at a first portion 15 *(e.g.,* a pivot point) of the housing portion 14 such that the lever 12 pivots between the first lever position to the second lever position about a pivot axis that extends through the pivot point 15, and the pivot axis may be parallel to the Y-axis of the reference coordinate system of Fig. 23E. In particular, the first portion of the lever 12 may be a pair of aligned bosses that are received into corresponding cylindrical internal walls that are each formed on a corresponding interior portion of the housing portion 14 at the pivot point 15 of the housing portion 14. A portion of the lever 12 may contact a portion of the housing portion 14 when the lever 12 is in the first lever position to prevent the lever 12 from overextending beyond the first lever position. A first end of a spring 55 may be coupled to a second portion 86 of the lever 12 and a second end of the spring 55 may be coupled to a portion 88 of the interior portion of the housing portion 14 such that the lever 12 is biased into the first lever position by the spring 55.

Referring to Figure 23A, the device 400 includes the shaft 18 that extends from a proximal end 20 to a distal end 22 along a shaft axis 19, and the shaft 18 may be fixed relative to the housing portion 12. However, one or more portions of the shaft 18 may be rotatably coupled to a portion of the housing portion 14 such that the shaft 18 rotates relative to a housing portion 14 about a portion of the shaft axis 19. The shaft axis 19 may have any shape or combination of shapes. As illustrated in Figure 23A, one or more portions of the shaft 18 may be non-linear. For example, an embodiment of the device 400 includes a first portion 18a of the shaft 18 that may be linear and may extend along a first portion 19a of the shaft axis 19 from the distal end 22 of the shaft 18 towards the proximal end 20 of the shaft 18 to an intermediate point. The first portion 18a of the shaft 18 may form an acute angle with the X-axis of the reference coordinate system of Fig. 23A, and the first portion 19a of the shaft axis 19 may extend along the X-Z plane of the reference coordinate system of Fig. 23A. A second portion 18b of the shaft 18 may extend along a non-linear second portion 19b of the shaft axis 19, and may extend from the intermediate point to the proximal end 20 of the shaft 18. The second portion 19b of the shaft axis 19 may be curved, and may have the shape of a segment of a circle when viewed along the Y-axis of the reference coordinate system of Fig. 23A. The shaft 18 may be rigid, but in other embodiments, the shaft 18 may be flexible or may have one or more portions that are flexible. In other embodiments, the shaft axis 19 may be linear from the proximal end 20 to the distal end 22.

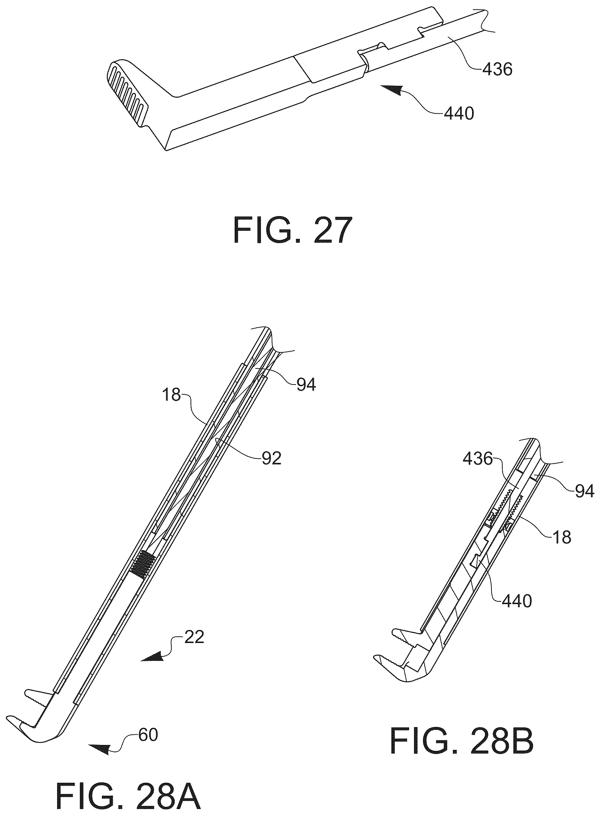

With reference to the cross-sectional view of Fig. 28A, the shaft 18, or one or more portions of the shaft 18, may have the general shape of an elongated hollow tube having an interior surface 92 that defines an interior portion 94 that extends from the proximal end 20 to the distal end 22 of the shaft 18. The shaft 18 and the interior surface 92 may have any suitable cross-sectional shape or combination of shapes normal to the shaft axis 19. For example, the shaft 18 may have the general shape of an elongated cylinder, and the interior surface 92 may have a circular cross-sectional shape when viewed normal to the shaft axis 19.

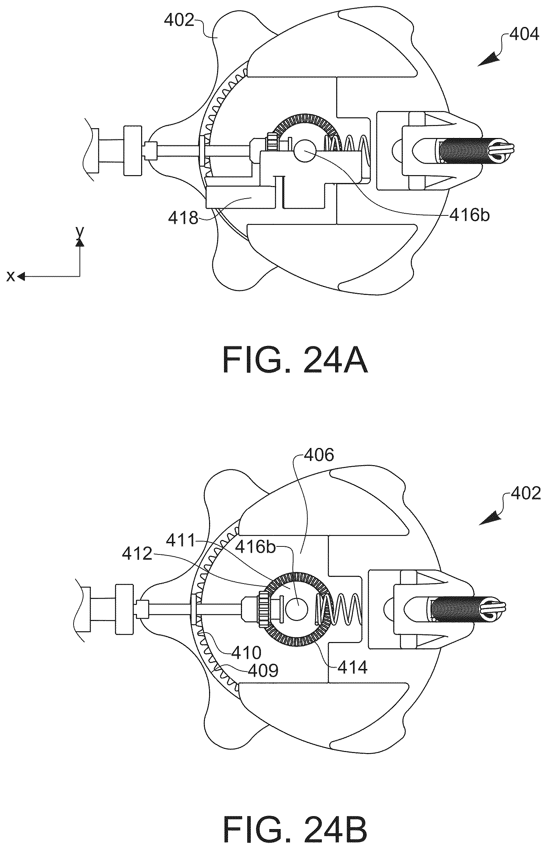

The device 400 may include a rotation assembly 402 may be coupled to the jaw assembly 60 to selectively rotate the jaw assembly 60 relative to the shaft 18. The rotation assembly 402 may include a rotation knob 404 that may be rotatably coupled to a portion of the housing portion 14. As illustrated in Figs. 24A to 24C, in which the housing portion 14 is omitted for clarity, the rotation knob 404 may include a disc-shaped body portion 406 that may be symmetrically formed about a knob rotation axis 405 that may extend in any suitable direction, such as parallel to the Z-axis of the reference coordinate system of Fig. 24A. The body portion 406 may have a plurality of notches 408 formed along a perimeter edge of the body portion 406, and the notches 408 are configured to facilitate the rotation of the rotation knob 404 by a finger of a user. A circumferential ridge 409 may be formed on an underside of the body portion 406 , and the circumferential ridge 409 may be symmetrically formed about the knob rotation axis 405. A plurality of locking teeth 410 may be disposed along the circumferential ridge 409.

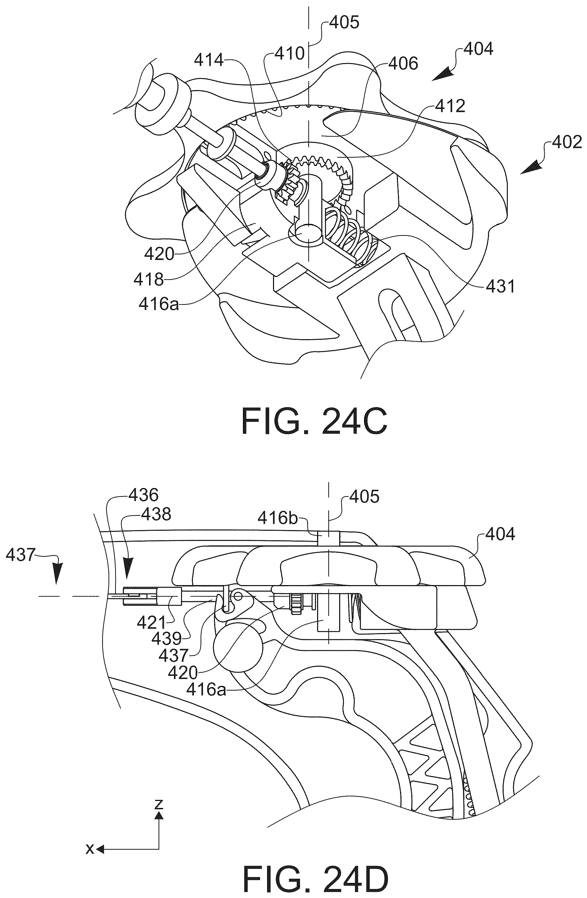

A cylindrical projection 412 may extend from a portion of the underside of the body portion 406, and the cylindrical portion 412 may extend along the knob rotation axis 405. The cylindrical portion 412 may have any suitable diameter, and may have a diameter that is less than a diameter of the circumferential ridge 409. A first portion of an elongated stem 416a may extend from a lower surface 411 of the cylindrical portion 412, and the first portion of the stem 416a may extend along the knob rotation axis 405. A second portion of the stem 416b may extend from an upper surface of the cylindrical portion 412, and the second portion of the stem 416b may also extend along the knob rotation axis 405. The first portion of the stem 416a may be coupled to a first portion of the housing portion 14, and the second portion of the stem 416b may be disposed within an aperture of a retainer housing 418, which is illustrated in Figs. 24A and 24C in which half of the retainer housing 418 is omitted for clarity. The retainer housing 418 may be directly or indirectly coupled to a second portion of the housing portion 14 such that the rotation knob 404 may rotate about the first portion of the stem 416a and the second portion of the stem 416b (i.e., about the knob rotation axis 405).

A lower surface 411 of the cylindrical portion 412 may have a plurality of drive teeth 414 that are disposed along a circumferential edge of the lower surface 411. The plurality of drive teeth 414 engage the teeth of a pinion gear 420 that is secured to or coupled to a portion of a transmission rod 460 that will be described in more detail in a following section. As illustrated in Fig. 24D, the pinion gear 420 has a rotational axis 421 that may be normal to the knob rotation axis 405, and the rotational axis 421 of the pinion gear 420 may be aligned with the direction of a longitudinal axis 467 of the transmission rod 460 and/or the direction of displacement of a push rod 36 (that will be described in more detail) when the lever 12 is pivoted from the first rod position (which is illustrated in Figure 13 of the embodiment of the device 300) to the second lever position, (illustrated in Figures 23A). Thus, when the rotation knob 404 is rotated in a first rotational direction about the knob rotation axis 205, the pinion gear 420 also rotates in a first rotational direction about the rotational axis 421 of the pinion gear 420, and when the rotation knob 404 is rotated in a second rotational direction about the knob rotation axis 205, the pinion gear 420 also rotates in a second rotational direction about the rotational axis 421 of the pinion gear 420.

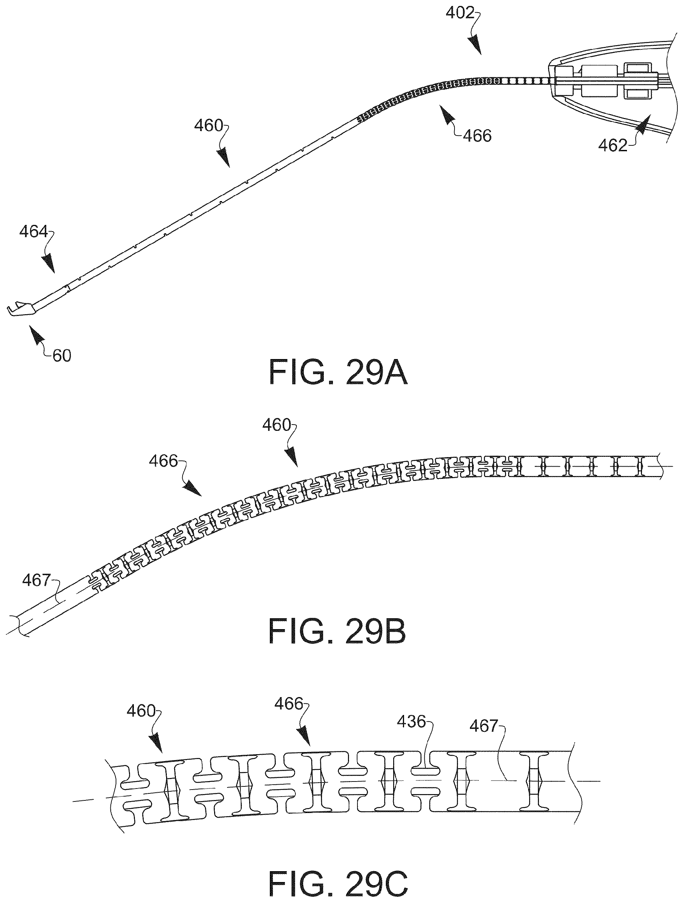

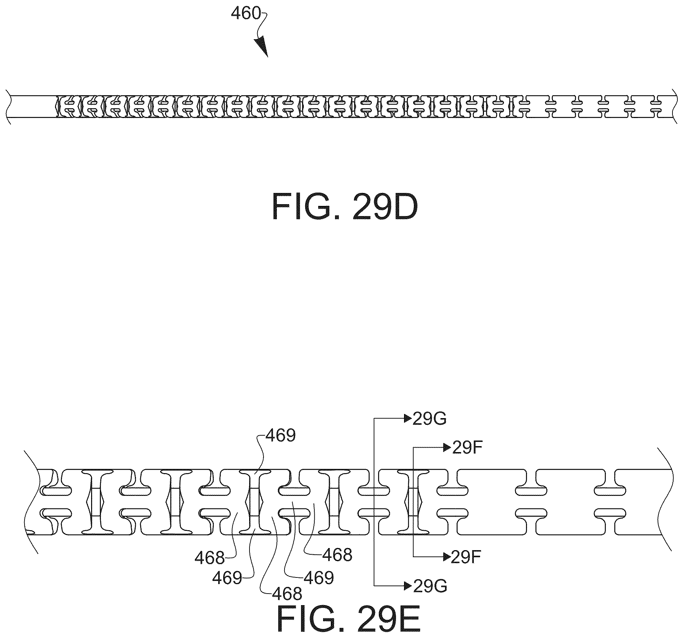

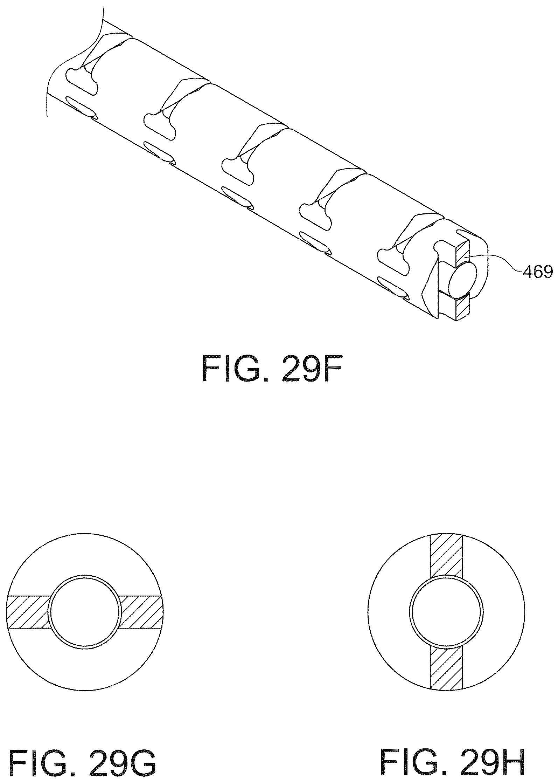

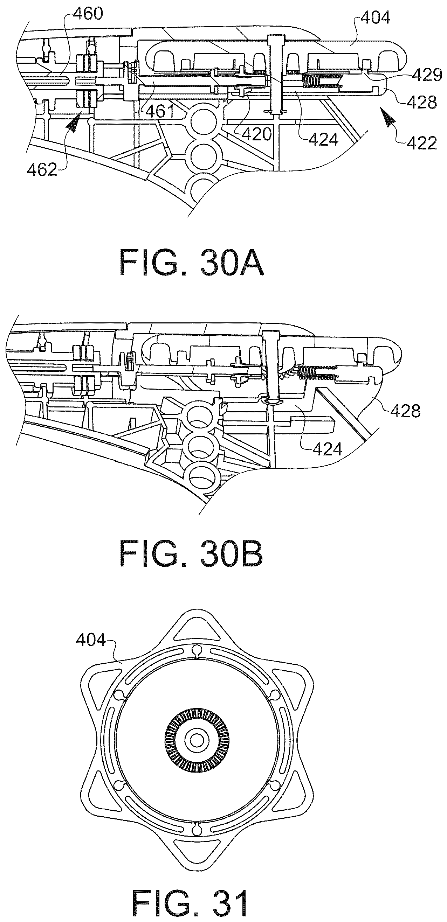

The rotation assembly 402 may further include the transmission rod 460 that may be coupled with the pinion gear 420, as illustrated in the side view of Fig. 29A in which a portion of the housing portion 12 and the shaft 18 are omitted for clarity. The transmission rod 460 is elongated and at least a portion of the transmission rod 460 extends through the interior portion 94 of the shaft 18. The transmission rod 460 may extend along a longitudinal axis 467 that is aligned with the non-linear shaft axis 19 from a proximal end 462 to a distal end 464 (illustrated in Fig. 30A). Thus, the longitudinal axis 467 of the transmission rod 460 may be aligned with the shaft axis 19, and thus one or more portions of the longitudinal axis 467 of the transmission rod 460 may be non-linear, and may have a curved shape.

The transmission rod 460 may be coupled to the pinion gear 420 in any suitable manner such that a user may rotate the rotation knob 404 to rotate the transmission rod 460. For example, as illustrated in Fig 33 and the cross-sectional view of Fig. 30A, the pinion gear 420 may be coupled to a shaft portion 461 that extends along the rotational axis 421 of the pinion gear 420 (and along a proximal portion of the longitudinal axis 467 of the transmission rod 460), and a distal end of the shaft portion 461 is coupled or secured to the proximal end 462 of the transmission rod 460 such that a rotation of the shaft portion 461 (a) in a first rotational direction results in a rotation of the transmission rod 460 (about the longitudinal axis 467) in the first rotational direction, and (b) in a second rotational direction results in a rotation of the transmission rod 460 (about the longitudinal axis 467) in the second rotational direction. The distal end 464 of the transmission rod 460 may be coupled to the proximal end of the jaw assembly 60 such that (a) the rotation of the transmission rod 460 in the first direction results in a rotation of the jaw assembly 60 in the first direction (relative to the fixed distal end 22 of the shaft 18) and that (b) the rotation of the transmission rod 460 in the second direction results in a rotation of the jaw assembly 60 in the second direction (relative to the fixed distal end 22 of the shaft 18). In some embodiments, the proximal end 66 of the first jaw 62 (see Fig. 17A) may be fixedly coupled to the distal end 464 of the transmission rod 460 such that (a) the rotation of the transmission rod 460 in the first direction results in the rotation of the first jaw 62 (and the second jaw 64 that is slidably coupled to the first jaw) in the first direction about the first jaw axis 70 (relative to the fixed distal end 22 of the shaft 18) and (b) the rotation of the transmission rod 460 in the second direction results in the rotation of the first jaw 62 (and the second jaw 64 that is slidably coupled to the first jaw) in the second direction about the first jaw axis 70 (relative to the fixed distal end 22 of the shaft 18).

Because one or more sections of the shaft 18 may be non-linear, one or more portions of the transmission rod 460 may be capable of bending to conform to the shape of the non-linear portion of the shaft 18 while still transmitting torque applied at the proximal end 462 of the transmission rod 460 to the distal end 464 of the transmission rod 460. For example, a portion (or more than one portion) of the transmission rod 460 may include the hinge portion 144 of Figs. 11A to 11C. A further embodiment of the hinge portion 466 is illustrated in Figs. 29A to 29E. The hinge portion 114 may include a plurality of disc members 468 that are offset from adjacent disc members 468 along the longitudinal axis 467, and the disc members 468 may be offset by equal distances or substantially equal distances along the hinge portion 466. Each disc member 468 may have a circular diameter that may be equal to the diameter of the transmission rod 460. Adjacent disc members 468 may be coupled by hinge plates 469 that are disposed between (and extend between) inner -facing surfaces of adjacent disc members 468 along the longitudinal axis 467. Each of the hinge plates 469 may be planar and may extend in a plane that is aligned with the longitudinal axis 467 and is normal to the plane of the adjacent disc members 468, and each hinge plate 469 may extend through (or be symmetrical about) the longitudinal axis of the transmission rod 460. The hinge plates 469 may alternate in orientation by 90° increments along the longitudinal axis of the transmission rod 460, as illustrated in the cross-sectional views of Fig. 29G and Fig. 29H. In some embodiments, the transmission rod 460 may include a rod aperture 470 along the longitudinal axis 467 of the transmission rod 460, and the rod aperture 470 may extend from the proximal end 462 to the distal end 464 of the transmission rod 460. A portion of the push rod 36 may extend through the rod aperture 470 such that the transmission rod 460 may rotate independently of the push rod 36 and the push rod 36 may displace longitudinally relative to the transmission rod to displace the second jaw 64 relative to the first jaw 62 as previously described.

As illustrated in the cross-sectional view of the device 400 of Fig. 23I, in which a portion of the housing portion 14 is omitted for clarity, the rotation assembly 402 may further include a locking assembly 422 that may selectively engage a portion of the rotation knob 404 to prevent rotation of the rotation knob 404 relative to the housing portion 14. The locking assembly 422 includes a locking lever 423 having an elongated arm portion 427 and a head portion 424 disposed at an end portion of the arm portion 427, the head portion 424 being configured to engage a portion of the rotation knob 404.

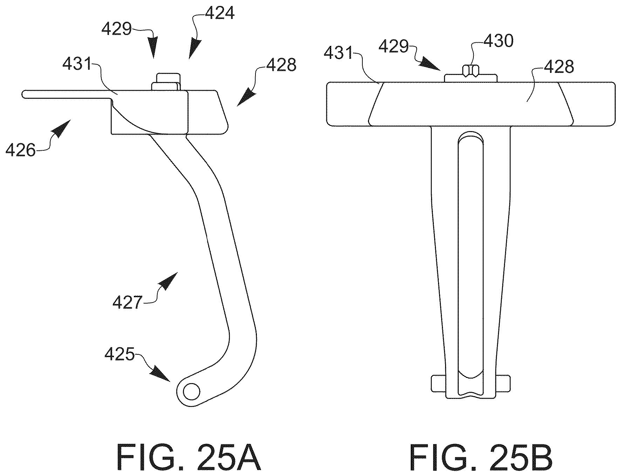

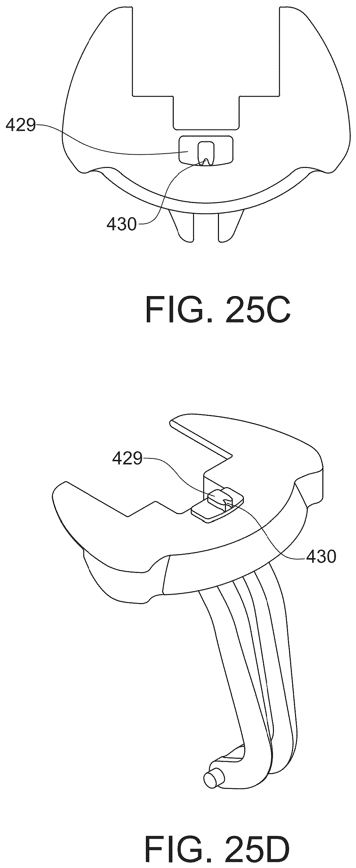

In particular, as illustrated in Figs. 25A to 25D, the arm portion 427 extends from a first end 425 to a second end 426, and the second end 426 is coupled to the head portion 424. The first end 425 is pivotably coupled to a portion of the housing portion 14 such that the locking lever 423 is pivotable from a first engaged position (illustrated in Fig. 23I) to a second disengaged position (not shown).

The head portion 424 includes a plurality of surfaces that cooperate to form a button portion 428, and a portion of the head portion 424 extends through an aperture through the housing portion 14 such that all or a portion of the button portion 428 is disposed external to the housing portion 14. So configured, one or more fingers of the user may apply inward pressure to the button portion 428 to pivot the locking lever 423 from the first engaged position to the second disengaged position. A locking protrusion 429 is disposed on an upper surface 431 of the head portion 424, and the locking protrusion 429 includes a notch 430 (or two or more notches 430) that is adapted to receive one (or more) of the plurality of locking teeth 410 of the body portion 406 of the rotation knob 404 when the locking lever 423 is in the first engaged position, thereby preventing the rotation knob 404 from rotating about the knob rotation axis 405. When the locking lever 423 is pivoted from the first engaged position to the second disengaged position, the locking protrusion 429 is displaced away from the plurality of locking teeth 410 of the body portion 406 of the rotation knob 404 such that none of the plurality of locking teeth 410 are disposed within the notch 430, thereby allowing the rotation knob 404 to rotate about the knob rotation axis 405.

The locking assembly 422 also includes a spring 431, such as a coil spring. A first portion of the spring 431 may be disposed in a recess formed in the head portion 424, and a second portion of the spring 431 may be disposed within a recess formed in a portion of the retainer housing 418 such that the head portion 424 is biased away from the portion of the retainer housing 418, thereby biasing the locking lever 423 in the first engaged position.

In the embodiment of the device 500 illustrated in Figs. 32A to 32D, an embodiment of the locking assembly 422 (illustrated in the cross-sectional view of Figs. 30A and 30B) does not include the arm portion 427 or the locking lever 423, but may include the head portion 424. The head portion 424 may include the locking protrusion 429 disposed on the upper surface 431 of the head portion 424. The locking protrusion 429 may include a single post 469, that may have any shape or combination of shapes, such as a cylindrical shape. The post 469 may be adapted to engage one or two (or more) of the plurality of locking teeth 410 of the body portion 406 of the rotation knob 404 when the head portion 424 is in the first engaged position, thereby preventing the rotation knob 404 from rotating about the knob rotation axis 405. When the head portion 424 is linearly displaced from the first engaged position to the second disengaged position, the locking protrusion 429 is displaced away from the plurality of locking teeth 410 of the body portion 406 of the rotation knob 404 such that none of the plurality of locking teeth 410 are engaged by the post 469, thereby allowing the rotation knob 404 to rotate about the knob rotation axis 405. A first portion of the spring 431 may engage a portion of the head portion 424 such that the head portion 424 is biased into the first engaged position.

Referring to Figure 24D, the device 400 (and a further embodiment of the device 500 illustrated in Figs. 32A to 32D) also includes a push rod 436 that may be similar to the push rod 36 of the device 300. The push rod 436 may extend along a push rod axis 437 from a proximal end 438 to a distal end 440 (illustrated in Fig 27, in which the shaft 18 and a first jaw 462 are omitted for clarity). The push rod 436 is elongated and at least a portion of the push rod 436 extends through the interior portion 94 of the shaft 18, as illustrated in Fig. 28B. The proximal end 438 of the push rod 436 is coupled to a portion of the lever 12 such that when the lever 12 is in the first lever position, the push rod 436 is in a first rod position, as illustrated in Figure 15. When the lever 12 is pivoted to the second lever position, (illustrated in Figures 24D) the push rod 436 is in a second rod position in which the distal end 440 of the push rod 436 (and the entire push rod 436) is displaced distally from the first position. The push rod 436 may translate linearly or substantially linearly from the first rod position to the second rod position along (or substantially along) the push rod axis 437, and the push rod axis 437 may extend parallel to (or substantially parallel to) or along the non-linear shaft axis 19.

The proximal end 438 of the push rod 436 may be coupled to the portion of the lever 12 in any suitable manner. For example, a disk-shaped projection 437 may extend radially from the push rod 436 at or adjacent to the proximal end 438, and the projection 437 may be disposed within a notch 439 formed in the portion of the lever 12. Thus, a first surface of the portion of the lever 12 forming the notch 439 may contact a first portion of the projection 437 to displace the push rod 436 from the first rod position to the second rod position when the lever 12 pivots from the first lever position to the second lever position, and a second surface of the portion of the lever 12 forming the notch 439 may contact a second portion of the projection 437 to displace the push rod 436 from the second rod position to the first rod position when the lever 12 pivots from the second lever position to the first lever position.

Various advantages of a probe device and a grasping device have been discussed above. Embodiments discussed herein have been described by way of example in this specification. It will be apparent to those skilled in the art that the foregoing detailed disclosure is intended to be presented by way of example only, and is not limiting. Various alterations, improvements, and modifications will occur and are intended to those skilled in the art, though not expressly stated herein. These alterations, improvements, and modifications are intended to be suggested hereby, and are within the scope of the claimed invention. The drawings included herein are not necessarily drawn to scale. Additionally, the recited order of processing elements or sequences, or the use of numbers, letters, or other designations therefore, is not intended to limit the claims to any order, except as may be specified in the claims. Accordingly, the invention is limited only by the following claims and equivalents thereto.

## Claims

1. A grasping device (10, 200, 300, 400, 500) for use in a surgical procedure, the grasping device (10, 200, 300, 400, 500) comprising:
a housing portion (12);
an actuator lever (16) pivotably coupled to a first portion (15, 18a, 19a, 24a, 58) of the housing portion (12) and displaceable between a first lever position to a second lever position;
a shaft (18) that extends from a proximal end (20, 34, 38, 41, 42, 46, 54, 62, 66, 68, 74, 82, 90, 98, 438, 462) to a distal end (22, 36, 40, 42, 48, 56, 64, 68, 76, 84, 92, 101, 440, 464) along a shaft axis (19, 24), wherein one or more portions of the shaft (18) are coupled to a second portion (18b, 19b, 21, 24b, 66, 86) of the housing portion (12), wherein the shaft axis (19, 24) is non-linear, the shaft (18) having one or more interior surfaces (13, 28) that defines an interior portion (94) extending from the proximal end (20, 34, 38, 41, 42, 46, 54, 62, 66, 68, 74, 82, 90, 98, 438, 462) to the distal end (22, 36, 40, 42, 48, 56, 64, 68, 76, 84, 92, 101, 440, 464) of the shaft (18);
an elongated push rod (36, 436) extending from a proximal end (20, 34, 38, 41, 42, 46, 54, 62, 66, 68, 74, 82, 90, 98, 438, 462) to a distal end (22, 36, 40, 42, 48, 56, 64, 68, 76, 84, 92, 101, 440, 464), wherein a portion (80, 88, 96) of the push rod (36, 436) is disposed within the interior portion (94) of the shaft (18), and wherein a proximal end (20, 34, 38, 41, 42, 46, 54, 62, 66, 68, 74, 82, 90, 98, 438, 462) of the push rod (36, 436) is coupled to a first portion (15, 18a, 19a, 24a, 58) of the actuator lever (16) and such that when the lever (16) is in the first lever position, the push rod (36, 436) is in a first rod position and when the lever (16) is pivoted to the second lever position, the push rod (36, 436) is in a second rod position in which the distal end (22, 36, 40, 42, 48, 56, 64, 68, 76, 84, 92, 101, 440, 464) of the push rod (36, 436) is displaced distally from the first position; and
a jaw assembly (60, 62) disposed at or adjacent to the distal end (22, 36, 40, 42, 48, 56, 64, 68, 76, 84, 92, 101, 440, 464) of the shaft (18), the jaw assembly (60, 62) comprising:
a first jaw (62, 64, 462) fixedly coupled to a portion (80, 88, 96) of the shaft (18) at or adjacent to the distal end (22, 36, 40, 42, 48, 56, 64, 68, 76, 84, 92, 101, 440, 464) of the shaft (18); and
a second jaw (64) coupled to the distal end (22, 36, 40, 42, 48, 56, 64, 68, 76, 84, 92, 101, 440, 464) of the push rod (36, 436) such that the second jaw (64) is displaceable relative to the first jaw (62, 64, 462) and such that in the first rod position, the jaw assembly (60, 62) is in a first position and in the second rod position, the jaw assembly (60, 62) in in a second position.

2. The grasping device (10, 200, 300, 400, 500) of claim **1,** further comprising a rotation assembly (402) coupled to a portion (80, 88, 96) of the jaw assembly (60, 62) to selectively rotate the jaw assembly (60, 62) relative to the shaft (18).

3. The grasping device (10, 200, 300, 400, 500) of claim 2, wherein the rotation assembly (402) includes a rotation knob (32, 106, 404) rotatably coupled to a third portion (18c, 19c, 24c, 39) of the housing portion (12), the rotation knob (32, 106, 404) comprising:
a disc-shaped body portion (406) that rotates about a knob rotation axis (205, 405); and
a projection (102, 437) extending from a portion (80, 88, 96) of an underside of the body portion (406) along the knob rotation axis (205, 405).

4. The grasping device (10, 200, 300, 400, 500) of claim 3, wherein a lower surface (411) of the projection (102, 437) includes a plurality of drive teeth (414) that are disposed along a portion (80, 88, 96) of the lower surface (411) of the projection (102, 437).

5. The grasping device (10, 200, 300, 400, 500) of any one of claims 2 to 4, the rotation assembly (402) further comprising a transmission rod (460) that extends from a proximal end (20, 34, 38, 41, 42, 46, 54, 62, 66, 68, 74, 82, 90, 98, 438, 462) to a distal end (22, 36, 40, 42, 48, 56, 64, 68, 76, 84, 92, 101, 440, 464) along a longitudinal axis (467), the proximal end (20, 34, 38, 41, 42, 46, 54, 62, 66, 68, 74, 82, 90, 98, 438, 462) of the transmission rod (460) being coupled to a gear (76) that engages a portion (80, 88, 96) of the plurality of drive teeth (414) of the projection (102, 437) of the rotation knob (32, 106, 404), and
wherein the distal end (22, 36, 40, 42, 48, 56, 64, 68, 76, 84, 92, 101, 440, 464) of the transmission rod (460) is coupled to the jaw assembly (60, 62) such that a rotation of the body portion (406) of the rotation knob (32, 106, 404) rotates the jaw assembly (60, 62) relative to the shaft (18).

6. The grasping device (10, 200, 300, 400, 500) of claim 5, wherein a portion (80, 88, 96) of the push rod (36, 436) extends through an aperture (26) formed through the transmission rod (460) such that the push rod (36, 436) is configured to displace relative to the push rod (36, 436) along the longitudinal axis (467) of the push rod (36, 436).

7. The grasping device (10, 200, 300, 400, 500) of claim 5 or 6, wherein the transmission rod (460) comprises a plurality of disc members (116, 468) that are offset from adjacent disc members (116, 468) along the longitudinal axis (467).

8. The grasping device (10, 200, 300, 400, 500) of any one of claims 3 to 7, wherein the rotation knob (32, 106, 404) is rotatably coupled to the third portion (18c, 19c, 24c, 39) of the housing portion (12) such that
(a) when the rotation knob (32, 106, 404) is rotated in a first rotational direction about the knob rotation axis (205, 405), the jaw assembly (60, 62) rotates in a first rotational direction about a first jaw axis (70), and
(b) when the rotation knob (32, 106, 404) is rotated in a second rotational direction about the knob rotation axis (205, 405), the jaw assembly (60, 62) rotates in a second rotational direction about the first jaw axis (70).

9. The grasping device (10, 200, 300, 400, 500) of any one of claims 4 to 8, wherein the plurality of drive teeth (414) are disposed along a circumferential edge of the lower surface (411) of the cylindrical projection (412).

10. The grasping device (10, 200, 300, 400, 500) of any one of claims 4 to 9, the rotation assembly (402) further comprising a locking assembly (120, 422) configured to selectively engage a portion (80, 88, 96) of the rotation knob (32, 106, 404) to prevent rotation of the rotation knob (32, 106, 404) relative to the housing portion (12).

11. The grasping device (10, 200, 300, 400, 500) of claim 10, wherein the locking assembly (120, 422) includes a head portion (424) that is displaceable relative to the housing portion (12) between a first position and a second position;
wherein in the second position, a portion (80, 88, 96) of the head portion (424) is configured to engage a portion (80, 88, 96) of the rotation knob (32, 106, 404) to prevent rotation of the rotation knob (32, 106, 404) relative to the housing portion (12).

12. The grasping device (10, 200, 300, 400, 500) of claim 10 or 11, wherein the locking assembly (120, 422) includes a locking lever (423) having an elongated arm portion (427) and the head portion (424) is disposed at an end portion (80, 88, 96) of the arm portion (427);
wherein the locking lever (423) is pivotable between a first position and a second position; and
wherein in the second position, a portion (80, 88, 96) of the head portion (424) is configured to engage a portion (80, 88, 96) of the rotation knob (32, 106, 404) to prevent rotation of the rotation knob (32, 106, 404) relative to the housing portion (12).

13. The grasping device (10, 200, 300, 400, 500) of any one of claims 1 to 12, wherein the shaft (18) comprises a first portion (15, 18a, 19a, 24a, 58), a second portion (18b, 19b, 21, 24b, 66, 86), and a third portion (18c, 19c, 24c, 39), and the third portion (18c, 19c, 24c, 39) of the shaft (18) extends from a distal end (22, 36, 40, 42, 48, 56, 64, 68, 76, 84, 92, 101, 440, 464) of the first portion (15, 18a, 19a, 24a, 58) to a proximal end (20, 34, 38, 41, 42, 46, 54, 62, 66, 68, 74, 82, 90, 98, 438, 462) of the second portion (18b, 19b, 21, 24b, 66, 86), and the third portion (18c, 19c, 24c, 39) extends along a corresponding portion (80, 88, 96) of the shaft axis (19, 24) that is curved.

14. The grasping device (10, 200, 300, 400, 500) of claim 13, wherein the first portion (15, 18a, 19a, 24a, 58) of the shaft (18) and the second portion (18b, 19b, 21, 24b, 66, 86) of the shaft (18) are each linear, but a portion (80, 88, 96) of the shaft axis (19, 24) corresponding to the first portion (15, 18a, 19a, 24a, 58) of the shaft (18) is not parallel to or coaxially-aligned with a portion (80, 88, 96) of the shaft axis (19, 24) corresponding to the second portion (18b, 19b, 21, 24b, 66, 86) of the shaft (18).
